# EUROPÄISCHE PATENTANMELDUNG

(11) **EP 1 764 371 A1**
(43) Veröffentlichungstag der Anmeldung: **21.03.2007**
(21) Anmeldenummer: 05009012.5
(22) Anmeldetag: 25.04.2005
(51) Int. Cl.: C07K 16/24, C07K 14/525, C07K 14/715, C07K 14/47, A61K 38/19, A61K 38/17, A61K 39/395

(54) **Agonistische Antikörper, welche an den LT-Beta-Rezeptor binden und dadurch Adipositas assoziierte Phänotypen modulieren, und deren Verwendung in der Therapie**

(71) Anmelder: Xantos Biomedicine AG, 81377 München (DE)
(72) Erfinder: Link, Dieter, Dr., 82152 Planegg (DE)

(57) **Zusammenfassung**

Die Erfindung betrifft die Verwendung eines Lymphotoxin-β-Rezeptor aktivierenden Agenz, vorzugsweise eines Lymphotoxin-β-Rezeptor-Antikörpers (anti-LT-β-R-AK), zur Herstellung einer pharmazeutischen Zusammensetzung zur Vorbeugung und/oder Behandlung von Adipositas und/oder von Adipositas-assoziierter Diabetes (Typ II) und/oder Metabolischem Syndrom und/oder anderen Adipositas-assozüerten Krankheiten.

## Beschreibung

### Technische Beschreibung der Erfindung

Die Erfindung betrifft Agonisten des LT-β-Rezeptors sowie deren Verwendung zur Aktivierung des Lymphotoxin-β-Rezeptor-Signalwegs, zur Interferenz und / oder Modulation von Adipozytendifferenzierung und zur Anwendung in Adipositas und/oder Metabolischem Syndrom. Die Erfindung betrifft insbesondere Agonisten, vorzugsweise Antikörper, welche durch rezeptorvermittelte Signaltransduktion die Differenzierung von Prä-Adipozyten zu reifen Adipozyten, und/oder die Einlagerung von Lipidmolekülen in differenzierenden oder reifen Adipozyten hemmen. Die Erfindung betrifft ebenfalls aktivierende Epitope auf Lymphotoxin-β-Rezeptoren, durch deren Wechselwirkung mit einem Agonisten, wie einem Antikörper der Rezeptor zur Signaltransduktion gebracht wird. Insbesonders betrifft die Erfindung Antikörper, die gegen den Lymphotoxin-β-Rezeptor gerichtet sind und die als Lymphotoxin-β-Rezeptor aktivierende Agenzien agieren. Diese Aktivierung kann vorzugsweise allein oder auch in Kombination mit anderen Lymphotoxin-β-Rezeptor aktivierenden Agenzien (Agonisten) stattfinden. Weiterhin betrifft die Erfindung pharmazeutische Zusammensetzungen und deren Verwendung zur Aktivierung des Lymphotoxin-β-Rezeptor-Signalwegs, zur Interferenz und / oder Modulation von Adipozytendifferenzierung, insbesondere zur Behandlung von Adipositas.

### Hintergrund der Erfindung

Adipositas (Fettleibigkeit) und Typ II Diabetes sind zwei der derzeit wichtigsten Stoffwechselerkrankungen, die gerade in der westlichen Welt zunehmend ein ernstes Problem darstellen. Femer sind gerade auch beim Typ II Diabetes inzwischen Tendenzen zu einer Pandemie zu beobachten.

Adipositas ist ein Zustand, der durch eine übermäßige Ansammlung von Fettgewebe im Körper gekennzeichnet ist. Wesentlich für die Bildung von Fettgewebe und damit zur Ausprägung eines fettleibigen Phänotyps sind die Adipozyten, d.h. diejenigen Zellen, welche das Fettgewebe des Körpers bilden. Nach neuerem Verständnis ist die Adipositas eine chronische Gesundheitsstörung, die sowohl auf einer polygenetischen Veranlagung beruht als auch durch Umweltfaktoren bedingt ist und mit einer hohen Begleit- und Folgemorbidität einhergeht. Hierbei ist Adipositas mit dem signifikanten Risiko insbesondere der Entwicklung eines Typ II Diabetes verbunden. Daher ist für Adipositas ein langfristiges Behandlungs- und Betreuungskonzept unerlässlich.

Das Vorliegen von Fettleibigkeit, Fettsucht bzw. Adipositas kann als Übergewichtigkeit oder (klinische) Adipositas im engeren Sinne klassifiziert werden. Sie kann über den so genannten "Body Mass Index" (BMI) definiert werden. Der BMI beschreibt das Verhältnis des Körpergewichtes zur Körpergröße. Der BMI wird nach der folgenden Formel berechnet: BMI = Körpergewicht (kg) geteilt durch Körpergröße zum Quadrat (m²).

Die Klassifizierung des BMI wird von der WHO (Weltgesundheitsorganisation) in Abhängigkeit von der Sterblichkeitsrate festgelegt. Der Normal-BMI (18,5-24,4 kg/m²) ist der Bereich mit dem geringsten relativen Sterblichkeitsrisiko. Die BMI-Werte 25 und 30 kg/m² sind die entsprechenden risikobezogenen Grenzwerte für Erwachsene für die Feststellung des Übergewichts bzw. der (klinischen) Adipositas.

Gemäß dieser Nomenklatur wird die Adipositas in unterschiedliche Grade aufgeteilt: Adipositas Grad I (BMI= 30-34,9), Adipositas Grad II (BMI=35-39,9), Adipositas Grad III (BMI größer 40) sowie der morbiden Adipositas/Super Adipositas (BMI größer 50). Übergewicht und Adipositas gehen einher mit Folge- bzw. Begleitkrankheiten (Komorbidität). Sie sind ein wesentlicher Risikofaktor für die Entwicklung eines Typ II Diabetes. Sie bedingen auch häufig soziale Isolation und Diskriminierung. Sie können psychische Störungen unterstützen bzw. auslösen und verringern somit die Lebenserwartung und Lebensqualität. Bei einem BMI größer 30 kg/m² ist die Mortalität (Sterblichkeit) um 1/3, bei 40 kg/m² um den Faktor 3, bei größer 40 kg/m² um den Faktor 3 -20, je nach Gewicht erhöht. Erhöht ist zum Beispiel auch das Risiko einer Krebserkrankung (Gynäkologische Karzinome (Endometrium-Karzinom 4-fach, Mamma- und Zervix-Karzinom 2-fach), Prostata-, Gallenblasen-, Kolon-Karzinom)). Der BMI als Bewertungsmaßstab für eine Adipositas lässt allerdings die Körpergewebszusammensetzung unberücksichtigt. Erhöhte Muskelmasse, Wassergehalt oder Knochenmasse können die Bewertung beeinträchtigen und müssen gegebenenfalls berücksichtigt werden. Es kann auch die Körperzusammensetzung aus den Kompartimenten - Fett und fettfreie Masse (Körperwasser wird hier zunächst nicht berücksichtigt) erfasst werden. Nach diesem Zweikompartimentmodell ist der Normalbereich gegeben durch eine Fettmasse < = 20% und eine fettfreie Körpermasse von > = 80%. Ein erhöhter Fettanteil wird als Adipositas beschrieben. Zudem weist eine Erhöhung der Extrazellulärmasse bzw. des Extrazellulärwassers im Verhältnis zur fettfreien Körpermasse auf eine Adipositas hin. Untersuchungen wie die Bioelektrische Impedanz-Analyse (BIA) oder die Infrarot-Spektrometrie (NIRI) sind geeignete und in der Praxis angewandte Methoden zur Ermittlung des Körperfettstatus. Ebenso ist die Fettverteilung für die Risikobewertung wichtig. Eine erhöhte abdominale Fettansammlung (zentraler oder androider Verteilungstyp) stellt ein deutlich höheres Risiko dar, als der periphere oder gynoide Verteilungstyp. Daher kann auch die "waist-to-hip"-Ratio (das Verhältnis von Taillenumfang (cm) zu Hüftumfang (cm)) in die Bewertung miteinbezogen werden. Bei Frauen (>0,8) bzw. Männer (>0,95) sind erhöhte Werte mit einer erhöhten Sterblichkeit assoziiert.

Da Adipositas als wesentlicher Risikofaktor für die Entwicklung eines Typ II Diabetes angesehen werden muss, ist die Diagnostik und Therapie der Adipositas bereits im frühen Kindesalter die wichtigste Maßnahme, um zum Beispiel eine Entwicklung des pädiatrischen Typ II Diabetes zu vermeiden. Im Kindes- und Jugendalter kann analog zum Erwachsenenalter der BMI zur Beurteilung von Übergewicht und Adipositas verwendet werden. Da der BMI im Kindes- und Jugendalter entsprechend den physiologischen Änderungen der prozentualen Körperfettmasse von deutlichen alters- und geschlechtsspezifischen Besonderheiten beeinflusst wird, sollte man bei seiner Beurteilung Alter und Geschlecht berücksichtigen. Wegen der geringen Inzidenz von Adipositas-abhängigen Erkrankungen im Kindes- und Jugendalter und mangels ausreichender longitudinaler Untersuchungen zum Gesundheitsrisiko der Adipositas im Kindes- und Jugendalter gibt es im Gegensatz zu der Situation beim Erwachsenen keine festlegbaren Grenzwerte für das gesundheitsgefährdende Ausmaß der Körperfettmasse in diesem Altersbereich. Es kann aber bei der Definition von Übergewicht bzw. Adipositas im Kindes- und Jugendalter das BMI-Perzentil verwendet werden (gewonnen durch Extrapolierung), welches im Alter von 18 Jahren in einen BMI von 25 kg/m² (Übergewicht) bzw. 30 kg/m² (Adipositas) mündet. Es kann zum Beispiel das 90. bzw. des 97. alters- und geschlechtsspezifische Perzentil als Grenzwert zur Definition von Übergewicht bzw. Adipositas verwendet werden. Die BMI-Werte 25 und 30 kg/m² sind die entsprechenden risikobezogenen Grenzwerte für Erwachsene. Bei einem BMI von 25 kg/m² beträgt bei Männern zum Beispiel das relative Risiko für einen Typ II Diabetes 2,2 bei Frauen sogar 5,5.

Eine Behandlung des Übergewichts und der Adipositas hat entweder das Ziel, eine weitere Gewichtszunahme zu verlangsamen, bevorzugt aber zu verhindern, oder aber ebenso bevorzugt eine Reduktion des Körpergewichts zu erreichen. Dementsprechendes gilt insbesondere für das Ausmaß des Fettgewebsanteils der zu behandelnden Individuen. Umgekehrtes gilt für die Behandlung einer Kachexie oder Lipodystrophie. Eine Behandlung der Übergewichtigkeit bzw. der Neigung zur Adipositas ist, angesichts des bereits in der Übergewichtigkeit erhöhten Risikos für Komorbiditäten, bevorzugt angezeigt für Individuen mit einem BMI größer gleich 25. Insbesondere gilt dies für den Fall, dass Komorbiditäten bereits manifest sind, hier insbesondere bei vorliegenden Anzeichen für die Entwicklung einer Insulinresistenz oder eines Typ II Diabetes. Besonders bevorzugt werden Individuen mit einem BMI größer 30 ohne bzw. mit Komorbiditäten behandelt, die als klinisch adipös bezeichnet werden. Ganz besonders bevorzugt gilt dies für Individuen mit einem höheren Grad der Adipositas.

Der Entwicklung einer Adipositas kann vorgebeugt werden bei Individuen mit normalem BMI oder einem BMI, der diese als übergewichtig qualifiziert, bevorzugterweise dann, wenn es Gründe gibt für die Annahme, dass diese Individuen mit erhöhter Wahrscheinlichkeit zu einer späteren Ausprägung der Adipositas (Erhöhung des BMI über 30 kg/m²), insbesondere assoziiert mit Komorbiditäten, neigen werden.

Solche Gründe können zum Beispiel in einer genetischen Prädisposition, einer nachhaltigen Störung des Lipid- oder Lipoprotein-Stoffwechsels (Dyslipidämien), einem der Adipositas förderlichen und eventuell nur schwer veränderbaren Lebensstil, und/oder einer Adipositas infolge anderer Erkrankungen gegeben sein. Dazu kann eine Diagnostik hilfreich sein, die es erlaubt, beispielsweise genetische Prädispositionen direkt oder indirekt, oder veränderte Gehalte oder Aktivitäten des Lymphotoxin-beta Rezeptors (LT-β-R) oder dessen natürlichen Liganden oder davon abhängiger Parameter zum Beispiel im Blut oder im Fettgewebe nachzuweisen.

Die im Stand der Technik bekannten Behandlungsverfahren für Adipositas beschränken sich im Wesentlichen auf eine strenge Diät, gekoppelt mit erhöhter Bewegung. Eine bekannte Therapie ist bisher nur über den Noradrenalin-Serotonin-Rezeptoruptake-Inhibitor "Sibutramin" (BASF, Ludwigshafen) oder den Lipasehemmer "Xenical" (Roche, Basel, Schweiz) möglich. Der Einsatzbereich dieser Medikamente ist jedoch auf Grund der auftretenden Nebenwirkungen/Komplikationen eingeschränkt. So ist Sibutramin beispielsweise nicht bei Bluthochdruck - einer typischen Komplikation bei Adipositas - einsetzbar. Zudem kann die Erkrankung nur symptomatisch behandelt werden.

Eine der häufigsten Ko-Morbiditäten der Adipositas ist Diabetes, speziell der Typ II Diabetes.

Beim Diabetes mellitus (allgemein auch als "Zuckerkrankheit" bekannt) handelt es sich um eine chronische Stoffwechselerkrankung, die durch einen erhöhten Blutzuckerspiegel gekennzeichnet ist. Unterschiedliche Ursachen der Erkrankung und auch verschiedene Krankheitsausprägungen erfordern die Unterscheidung von zwei Typen, dem Typ I und dem Typ II Diabetes.

Der Typ I Diabetes (früher: juveniler Diabetes) beginnt meist in der Jugend und entsteht durch eine immunologische Zerstörung der Inselzellen des Pankreas (= Bauchspeicheldrüse). Diese Inselzellen produzieren das Hormon Insulin, das für die Verwertung der Glukose aus der Nahrung verantwortlich ist. Durch die Zerstörung der Inselzellen kommt es zu einem absoluten Insulinmangel. Die Glukose aus der Nahrung kann nicht mehr metabolisiert werden, und der Blutzuckerspiegel steigt. Die Behandlung des Typ I Diabetes geschieht durch die Verabreichung von Insulin.

Der Typ II Diabetes (früher: Erwachsenen- oder Altersdiabetes) entwickelt sich in der Regel im höheren Lebensalter. Er ist dadurch gekennzeichnet, dass die Körperzellen, an denen das Insulin wirken soll, nicht mehr ausreichend auf Insulin reagieren. Dies kann unter anderem auf eine Resistenz von Adipozyten und auch Skelettmuskelzellen gegenüber Insulin zurückgeführt werden. Interessanterweise sind zumeist die älteren, reiferen Adipozyten resistent, während jüngere Adipozyten in der Regel keine Insulinresistenz aufweisen.

Eine solche Insulinresistenz wird als Folge anhaltend hoher Blutzucker- und Insulinspiegel gesehen, wie sie z.B. bei Übergewichtigen zu beobachten sind. Die Therapie des Typ II Diabetes erfolgt stufenweise: Zunächst wird mit einer Diät versucht, den Blutzuckerspiegel allgemein zu senken. Sind die Diätmaßnahmen zur Behandlung nicht ausreichend, werden in der Folge Blutzucker senkende Medikamente und im fortgeschrittenen Stadium auch Insulin verabreicht.

Die Verabreichung von Insulin kann jedoch bei Patienten mit Typ II Diabetes aufgrund der Insulinresistenz in den Zielgeweben zu Hyperinsulinämie (erhöhter Insulinspiegel in Plasma, Serum) führen. Eine Therapie ist dann mittels Insulin nicht mehr möglich (Curr Diab Rep., Oktober 3 (5), Seite 378-85, (2003)).

Im Rahmen eines von der Anmelderin durchgeführten Projektes zur Identifizierung von Proteinfaktoren mit Relevanz für die Übergewichtigkeit, Adipositas oder Typ II Diabetes oder Kachexie, hat die Anmelderin das Protein TNFSF14, bevorzugt in einer trimeren Anordnung und in seiner löslichen Form als neuen Modulator der Adipozytendifferenzierung nachgewiesen.

Dieses Protein TNFSF14 entfaltet therapeutische Wirkung, bevorzugt durch eine direkte Einwirkung auf bestimmte Fettgewebe innerhalb eines Individuums oder auch indirekt, vermittelt durch andere Fettgewebskompartimente oder auch durch Wechselwirkung über andere Gewebe, die in die Regulation der Energie-Homöostase involviert sind.

Es hat sich femer gezeigt, dass dieses Protein TNFSF14 eine wesentliche Rolle bei der Behandlung von Adipositas spielt. Genauer gesagt, hat es sich herausgestellt, dass TNFSF14 in der Lage ist, die Differenzierung bzw. Funktion von Adipozyten zu hemmen. Dies ermöglicht erstmalig eine gezielte Behandlung von Adipositas.

Die Erklärung für den gefundenen Phänotyp des Proteins TNFSF14 ergibt sich aus der Eigenschaft von TNFSF14 als Rezeptor-Ligand, welcher bei Bindung Signaltransduktion auslösen kann:

Das Protein TNFSF-14 ist ein integrales Klasse-II-Membranprotein der Tumor-Nekrose-Faktor (TNF)-Liganden-Familie (Review in Cytokine & Growth Factor Reviews, 14, Granger and Ricert, Seite 289-296, (2003)). Es weist eine Länge von 240 Aminosäuren auf und enthält eine N-terminale zytosolische Domäne, eine Typ II Transmembrandomäne sowie eine C-terminale Rezeptorbindungsdomäne. Das lösliche Protein, abgeleitet von TNFSF14, ist 167 Aminosäuren lang und kann seinerseits die korrespondierenden Rezeptoren aktivieren. Diese Rezeptoraktivierung selbst kann in der Zelle u.a. Veränderungen auslösen, beispielsweise Zellüberleben oder entzündliche Prozesse induzieren oder modulieren.

Das Protein TNFSF14 ist ein Ligand für ein Mitglied der TNF-Rezeptor-Superfamilie, der auch als Lymphotoxin-beta Rezeptor (LT-β-R) bekannt ist. Weitere Rezeptoren sind der "herpes virus entry mediator" (HVEM) und DcR3.

Bisher wurde für dieses Liganden-Rezeptorsystem eine Beteiligung in immunologischen Prozessen (T-Zell-Proliferation, Zytokin-Sekretion, Arteriosklerose, "graft versus host disease") sowie eine Assoziation mit apoptotischen Prozessen gezeigt. Das Protein TNFSF14 stimuliert die Proliferation von T-Zellen und triggert die Apoptose von verschiedenen Tumorzellen. Bekannt ist auch die Prävention von TNF-alpha-mediierter Apoptose in Hepatozyten.

Im Rahmen der Arbeiten der Anmelderin konnte überraschenderweise zusätzlich hierzu eine Inhibition der Adipozyten-Differenzierung bzw. Hemmung der Lipideinlagerung in Adipozyten als Resultat der TNFSF14 vermittelten Aktivierung der LT-β-R-Signaltransduktion gezeigt werden.

Der LT-β-Rezeptor ist als ein Rezeptor der TNF-Familie bekannt (Crowe et al., Science, 264, Seite 707-710 (1994)). Der LT-β-Rezeptor bindet heteromere Lymphotoxinkomplexe (LT-α/β), die LT-α-Untereinheiten in Verbindung mit einem weiteren TNF-verwandten Polypeptid, dem Lymphotoxin-β (LT-β), beinhalten. Diese LT-α/β-Komplexe sind mebranassoziiert und weisen meistens eine LT-α1/β2-Stöchiometrie auf (Browning et al., Cell, 72, Seite 847 - 856 (1993), Browning et al., J. Immunol. 154, Seite 33 - 46 (1995)). In Analogie zu TNF-Rezeptoren (TNF-R) und anderen TNF-ähnlichen Rezeptoren wird angenommen, dass die Aktivierung des LT-β-R-Signalweg auftritt, wenn Rezeptoren auf der Zelloberfläche in nächster Nähe exprimiert werden (Crowe et al., Science, 264, S. 707-710 (1994)). Dieser Prozess ist bekannt als Rezeptorklusterbildung. Die TNF- und LT-Liganden sind multivalente Komplexe, die gleichzeitig an mehr als einen Rezeptor binden und auf diese Weise aggregieren können. Rezeptorklusterbildung als Mittel zur Rezeptoraktivierung in anderen Systemen ist ausführlich beschrieben worden, insbesondere für die Tyrosinkinase (Ullrich und Schlessinger, Cell, 61, Seite 203 - 212 (1990); Kolanus et al., Cell, 74, Seite 171 - 183 (1993)). Die Gabe von LT-α1/β2-Liganden und / oder LT-β-R-aktivierenden Agenzien kann deshalb die Klusterbildung und damit eine stromabwärts gelegene Signalgebung von LT-β-R-Molekülen auf der Oberfläche von Zielzellen induzieren. Der Signalweg durch LT-β-R ist bekannt dafür, dass er wie TNF-R unter anderem auch Reaktionswege aktiviert, die zu Toxizität und Zelltod in Tumorzellen führen kann. Diese Eigenschaft der LT-β-R-Aktivierung in Tumorzellen stellt auch die Basis für die in der Patentanmeldung EP 809510B1 beschriebene Verwendung von LT-β-R-aktivierenden Agenzien, einschliesslich spezifischer agonistischer anti-LT-β-R-Antikörper für die Behandlung und Reduzierung von Neoplasien dar. Ob sich eine solche Aktivierung allerdings auch toxisch auf normale Gewebe auswirkt, und deshalb der Rezeptor ein gutes Tumorzielmolekül darstellt, ist im Stand der Technik bisher ungeklärt.

Im Gegensatz zu den bisher beschriebenen Effekten der LT-β-R-Aktivierung und deren potentieller Verwendung in der Tumortherapie hat die Anmelderin aufgrund vorangegangener Experimente überraschenderweise gefunden, dass der LT-β-Signalweg nicht nur in den oben beschriebenen bekannten apoptotischen und entzündlichen Prozessen eine Rolle spielt, sondern auch eine Funktion in der Differenzierung von Adipozyten hat. So konnte gezeigt werden, dass Liganden, wie TNFSF14, aber auch LTa1β2 und LTa2β1, welche den Rezeptor aktivieren, ihrerseits die Fettzelldifferenzierung hemmen können. Da Rezeptoraktivierung auch durch agonistische Antikörper erzielt werden kann, beispielsweise durch solche, die den entsprechenden Rezeptor LT-β-R erkennen, können anstelle des natürlichen Liganden auch Agonisten, z.B. agonistische Antikörper, verwendet werden, welche den oben beschrieben LT-β-R gezielt und spezifisch aktivieren.

Die durch die Experimente der Anmelderin überraschenderweise gefundene Funktionalität des LT-β-R-Signalweges in der Differenzierung von Adipozyten bildet die Grundlage für eine alternative Behandlung von Adipositas und Adipositas-assoziierten Krankheiten.

Wie bereits oben beschrieben wurde, stellt die Behandlung von Adipositas und der mit Adipositas assoziierten Krankheiten ein enormes klinisches Problem dar. Bisherige Therapieansätze erfolgten und erfolgen derzeit nur symptomatisch entweder über veränderte Ernährung und, im Falle von Adipositas-assoziierter Diabetes, beispielsweise über die Verabreichung von PPAR-gamma Agonisten. Als Liganden für PPAR-gamma aktivieren diese den PPAR-gamma Transkriptionsfaktor, der eine Differenzierung der Adipozyten oder eine Aktivierung des Fettstoffwechsels in reifen Adipozyten bewirken kann. Nachteile solcher symptomatischen Behandlungen (u.a. der Diabetes) sind zudem das Auftreten von Folgeerscheinungen, wie beispielsweise kardiovaskulären Komplikationen, diabetischen Nephropathien, Neuropathien oder Retinopathien, die zusätzlich therapiert werden müssen. Alle bisher bekannten therapeutischen Ansätze haben zudem den folgeschweren Nachteil, dass diese nicht in der Lage sind, die Differenzierung bzw. Funktion von Adipozyten zu hemmen.

Eine Aufgabe der vorliegenden Anmeldung ist es daher, weitere Mittel und Wege zur Behandlung und / oder Vorbeugung von Adipositas, und damit auch zur Vorbeugung von Adipositas-assoziierter Diabetes (Typ II) bereitzustellen.

### Detaillierte Beschreibung der Erfindung

Erfindungsgemäß wird das beschriebene Problem durch die Bereitstellung von weiteren Mitteln und Wegen zur Behandlung und / oder Vorbeugung von Adipositas gelöst, durch von Substanzen, welche die Differenzierung bzw. Funktion von Adipozyten nachhaltig hemmen. Diese Substanzen zeichnen sich erfindungsgemäss durch ihre Fähigkeit aus, LT-β-R-vermittelte Signaltransduktion zu induzieren. Beispiele für Agentien, welche LT-β-R-vermittelte Signaltransduktion induzieren, sind die natürlichen Liganden oder homologe Liganden (oder spezies-fremde Liganden) von LT-β-R. Weil jedoch ein weiteres Problem bei der therapeutischen Verwendung von natürlichen Liganden des LT-β-R (wie beispielsweise TNFSF14) zur Behandlung von Adipositas und assoziierten Krankheiten deren effiziente Herstellung, sowie deren Verhalten im menschlichen Körper (Proteinstabilität, Pharmakologie, Halbwertszeit im Serum, Gewebeverteilung, unerwünschte Nebenreaktionen etc.) ist, werden erfindungsgemäss Agonisten, vorzugsweise Antikörper, welche LT-β-R erkennen und aktivieren, anstelle natürlicher Liganden verwendet. Die Verwendung von insbesondere Antikörpern, welche den Rezeptor LT-β-R erkennen und aktivieren, hat insofern Vorteile, als dass diese sowohl einfacher herzustellen sind, als auch aufgrund deren guten pharmakologischen Verhaltens kontrollierter im Patienten anzuwenden sind.

Das beschriebene Problem der Bereitstellung von Agenzien zur Behandlung und /oder Vorbeugung von Adipositas wird gelöst durch die erfindungsgemäßen Agonisten, wie Antikörper bzw. Derivate davon, die den Rezeptor LT-β-R erkennen und aktivieren. Diese agonistischen Antikörper und Zusammensetzungen, diese enthaltend, werden eingesetzt zur Behandlung von Adipositas und weiteren metabolischen Krankheiten, die mit gesteigerter oder aberranter Menge an Fettzellen assoziiert sind, indem der LT-β-R-Signalweg stimuliert wird.

Eine bevorzugte Ausführungsform der Erfindung beschreibt die Verwendung von wenigstens einem Antikörper, der gegen LT-β-R (anti-LT-β-R AK) gerichtet ist, zur Modulierung der Fettzelldifferenzierung, bevorzugt ist die Verwendung eines monoklonalen oder rekombinanten Antikörpers (anti-LT-β-R mAK). Eine weitere Ausführungsform der Erfindung beschreibt Antikörper oder Antikörperderivate, die gegen spezifische Epitope des Rezeptors, welche bei Bindung durch Antikörper die Aktivierung des Rezeptors verursachen, gerichtet sind.

Eine weitere Ausführungsform der Erfindung beschreibt Bindemoleküle (beispielsweise Affiline, Antikaline, Aptamere), die gegen solche Epitope des Rezeptors, welche bei Bindung durch Antikörper die Aktivierung des Rezeptors verursachen, gerichtet sind.

Die Erfindung stellt weiterhin ein neues Screening-Verfahren zur Selektion von LT-β-R aktivierenden Agenzien dar, wobei diese Agenzien u.a. Anti-LT-β-Antikörper,-Derivate, Bindeproteine und Bindemoleküle umfassen, und durch Bindung an mindestens ein aktivierendes Epitop des LT-β-R die Aktivierung des LT-β-R ermöglichen.

Der dabei verwendete Ansatz benutzt beispielsweise Antikörper gegen Epitope des Rezeptors, welche bei Bindung durch Antikörper die Aktivierung des Rezeptors verursachen und den Rezeptorsignalweg auslösen. Dieses kann entweder direkt durch Messung von Signaltransduktionsereignissen oder indirekt, z.B. durch Analyse der Modulation zellulärer Phänotypen, durch beispielsweise Inhibierung der Fettzeildifferenzierung gemessen werden.

Das verwendete Verfahren, um beispielsweise die agonistischen Eigenschaften und Anwendbarkeiten in der Modulation der Fettzelldifferenzierung von möglichen agonistischen Antikörper für LT-β-R zu testen, wird anhand von Beispielen beschrieben und umfasst vorzugsweise die folgenden Schritte:
1) Undifferenzierte humane Präadipozyten werden subkonfluent in Zellkulturschalen ausgesät und bis zur Postkonfluenz kultiviert .
2) Diese Zellen werden mit einem spezifischen Adipozyten-Differenzierungsubstanz ― Gemisch inkubiert, was im Normalfall zur messbaren Differenzierung in Adipozyten mit eingelagertem Fett führt.
3) Simultan werden die derart zur Differenzierung angeregten Zellen mit zu testenden Agenzien, wie Anti-LT-β-R-Antikörpern, -Antikörperpräparationen oder -Formulierungen inkubiert, bzw. vorzugsweise mit Kontrollantikörpern.
4) Die Messung der Differenzierung der Zellen erfolgt nach >= 8 Tagen mit einem Test, der zur Detektion von reifen Fettzellen geeignet ist (beispielsweise mit einem Lipidnachweistest, z.B. Nile Red Assay, wie in Beispiel 1 beschrieben).
5) Agenzien, welche die Fettzelldifferenzierung modulieren, werden dadurch erkannt, dass in diesen Zellen eine Abweichung der Test-Signalstärke stattfindet, welche mehr als eine einfache Standardabweichung beträgt, vorzugsweise eine mehr als 2-fache Änderung vom Signal, welches mit negativen Kontrollantikörpern erhalten wird.
6) Die Stärke der differenzierungsmodulierenden Aktivitäten der getesteten Agenzien, die im vorhergehenden Schritt positiv waren, wird durch serielle Verdünnungen und darauffolgende Bestimmungen dosisabhängiger Phänotypänderungen analysiert.

Dieses Verfahren kann an das Ziel, neue LT-β-R aktivierende Agenzien, z.B. Anti-LT-β-Antikörper, -Derivate, Bindeproteine und Bindemoleküle, zu identifizieren, moduliert und angepasst werden.

So können beispielsweise anstelle differenzierender Präadipozyten für das beschriebene Verfahren zur Generierung von differenzierungsmodulierenden Agenzien auch andere Zellen oder Zelllinien, welche den LT-β-R, natürlicherweise, in rekombinanter Form als Chimären-Rezeptor, oder entsprechende Epitop-haltige Moleküle exprimieren, verwendet werden. Bei der Verwendung solcher Zellen anstelle der Präadipozyten, kann der LT-β-R aktivierende Effekt von Agenzien beispielsweise über den Nachweis von spezifischen Signaltransduktionsereignissen in der Zelle festgestellt werden.

Ein anti-LT-β-R-Antikörper (oder eine Kombination von Antikörpern), der die Differenzierung von Adipozyten inhibiert oder moduliert, ist in dieser Versuchsanordnung ein LT-β-R aktivierendes Agenz. Diese Agenzien werden zur Herstellung einer pharmazeutischen Zusammensetzung zur Behandlung von Adipositas und weiteren metabolischen Krankheiten, die mit gesteigerter oder aberranter Menge an Fettzellen assoziiert sind, verwendet.

Wie oben ausgeführt betrifft die Erfindung ebenfalls die Verwendung von funktionellen Varianten der oben aufgeführten Antikörper zur Herstellung einer pharmazeutischen Zusammensetzung zur Behandlung von Adipositas und weiteren metabolischen Krankheiten, die mit gesteigerter oder aberranter Menge an Fettzellen assoziiert sind.

Gemäß einer bevorzugten Ausführungsform ist die Adipositas eine unkomplizierte Adipositas. Eine unkomplizierte Adipositas ist dadurch gekennzeichnet, dass noch keine Insulinresistenz ausgeprägt ist.

### Detaillierte Beschreibung der Erfindung

Die nachfolgende Beschreibung soll bevorzugte Ausführungsformen der Erfindung beispielhaft darlegen.

### Definitionen

*Der Begriff* "Adipozyten-Differenzierung" bezieht sich auf die Fähigkeit einer entsprechend determinierten Vorläuferzelle (Präadipozyt), sich auf geeignete Stimuli hin terminal zu differenzieren. Dabei wird ein spezifisches Genexpressionsprogramm initiiert, das phänotypisch unter anderem zu einer intrazellulären Akkumulation von Lipid führt. Eine derart differenzierter Adipozyt wird auch als reifer Adipozyt bezeichnet.

*Der Begriff* "Metabolisches Syndrom" bezieht sich auf die variable Kombination von klinischen Parametern und Symptomen, die u.a. mit Adipositas assoziiert sind. Dieses schliesst unter anderem häufig Adipositas und/oder Bluthochdruck und/oder hohe Cholesterinwerte und/oder Insulinresistenz ein.

Der Begriff "Epitop" (oder Antikörper-Bindestelle) ist definiert als die räumlich angeordnete chemische Struktur eines Antigens, die von einem Antikörper erkannt und gebunden wird.

Ein monoklonaler Antikörper (mAK) erkennt ein einziges Epitop, da alle Antikörper von einer produzierenden B-Zelle (= eine antikörperproduzierende Zelle) abstammen und demnach diesselbe Struktur aufweisen. Polyklonale Antikörper (pAK) sind Gemische verschiedener Antikörper, synthetisiert durch verschiedene B-Zellklone, welche verschiedene Epitope erkennen können, da sie unterschiedliche Antigenbindungsstellen aufweisen. Ein Epitop umfasst in der Regel mehrere Aminosäuren (beispielsweise 5-7 Aminosäuren), die sich in linearer Abfolge in der Primärzequenz des Antigens befinden können (= kontinuierliche Epitope oder lineare Epitope), oder aus verschiedenen, nicht unmittelbar verknüpften Aminosäuren des Antigens bestehen können (= diskontinuierliche Epitope, nicht-lineare Epitope).

*Der Begriff* "Fc-Domäne" eines Antikörpers bezieht sich auf einen Teil eines Moleküls, der die CH2-, CH3- und Hingeregionen umfasst, dem jedoch die Antigenbindestellen fehlen.

*Der Begriff* "LT-β-R aktivierendes Agenz" bezieht sich auf jedes Agenz, das in der Lage ist, Ligandenbindung an LT-β-R, Klusterbildung von LT-β-R auf der Zelloberfläche oder den LT-β-R-Signalweg zu verstärken, oder das Einfluß darauf ausüben kann, wie innerhalb der Zelle das LT-β-R-Signal interpretiert wird. Beispiele für LT-β-R aktivierende Agenzien sind TNFSF14, LT-a1/β2, lösliche anti-LT-β-R-Antikörper, kreuzverknüpfte anti-LT-β-R-Antikörper sowie multivalente anti-LT-β-R-Antikörper.

*Der Begriff* "LT-β-R-Signalweg" bezieht sich auf alle molekularen Reaktionen, die im Zusammenhang mit der Liganden-, Antikörper- oder Bindepartner-vermittelten Aktivierung des LT-β-R und den daraus resultierenden molekularen Reaktionen stehen.

*Der Begriff* "anti-LT-β-Rezeptor-Antikörper" ("anti-LT-β-R-AK") bezieht sich auf alle Antikörper, die wenigstens ein Epitop des LT-β-Rezeptors erkennen und daran binden.

*Der Begriff* "anti-LT-β-R-Antikörper, poly- oder monoklonal, kreuzverknüpfendes Agenz" bezieht sich auf alle Agenzien, die entweder kovalent oder nicht-kovalent anti-LT-β-R-Antikörper in Lösung binden können, so dass die Antikörper an der Oberfläche potentieller Zielzellen binden und dort Rezeptor-Signaltransduktion induzieren. Dieses schliesst auch Antikörper ein, welche die Rezeptoren aggregieren können, so dass die Antikörper an der Oberfläche potentieller Zielzellen binden und dort Rezeptorklusterbildung vervielfachen können.

*Der Begriff* "funktionelle Variante eines Antikörpers" bezieht sich erfindungsgemäss auf einen Antikörper und/oder Fragment, das im wesentlichen die biologische Funktion oder Funktionen des Antikörpers vermittelt. Im Falle der vorliegenden Antikörper kann es sich hierbei um die Fähigkeit handeln, die Lipideinlagerung in geeigneten Zellen zu inhibieren. Unter diesen Begriff fallen ebenfalls verschiedene Derivate von Antikörpern, insbesondere rekombinante, chimäre, humanisierte, oder anderweitig modifizierte Antikörper, welche die LT-β-R-Signaltransduktion induzieren.

*Der Begriff* "funktionelle Variante" schliesst auch "Nicht-Antikörper"-Proteine mit ähnlichen Bindeeigenschaften ein, beispielsweise Antikaline, Affiline, EinzeldomänenAntikörper und andere spezifische Bindeproteine.

*Der Begriff* "Bindungsprotein" oder "Bindungspeptid" bezieht sich auf eine Klasse von Proteinen, Peptiden oder Fragmenten, welche das jeweilige Molekül binden oder inhibieren, einschließlich, ohne Beschränkung, polyklonale oder monoklonale Antikörper, Antikörperfragmente und Proteingerüste, die gegen diese Proteine, Peptide oder Fragmente gerichtet sind.

*Der Begriff* "Aptamer" beschreibt Nukleinsäuren, die mit hoher Affinität an ein Polypeptid binden. Aptamere können durch Selektionsverfahren wie SELEX (siehe z.B. Jayasena, Clin. Chem., 45, Seite 1628 - 1650, (1999); Klug und Famulok, M. Mol. Biol. Rep., 20, Seite 97-107, (1994); US 5,582,981) von einem großen Pool unterschiedlicher Einzelstrang-RNA-Moleküle isoliert werden. Aptamere können auch in ihrer Spiegelform synthetisiert und selektiert werden, beispielsweise als das L-Ribonukleotid (Nolte et al., Nat. Biotechnol., 14, Seite 1116 - 1119, (1996); Klussmann et al., Nat. Biotechnol., 14, Seite 1112 - 1115, (1996)). Formen, die auf diese Weise isoliert wurden, haben den Vorteil, dass sie nicht durch natürlich vorkommende Ribonukleasen abgebaut werden und daher eine größere Stabilität aufweisen.

### Herstellung von anti-LT-β-R-Antikörpern zur Modulation der Differenzierung von Adipozyten

Das generelle Verfahren zum Herstellen eines Antikörpers oder Antikörperfragments wird mittels Verfahren durchgeführt, die dem Fachmann bekannt sind, beispielsweise durch Immunisieren eines Säugers, beispielsweise eines Kaninchens, mit dem entsprechenden Antigen, wobei, falls erforderlich, entsprechende Adjuvantien, beispielsweise Freunds Adjuvans und/oder Aluminiumhydroxidgele, verwendet werden können (siehe beispielsweise Diamond, B.A. et al., The New England Journal of Medicine, Seite 1344 - 1349, (1981)). Die polyklonalen Antikörper, die in dem Tier als Ergebnis einer immunologischen Reaktion gebildet werden, können nachfolgend aus dem Blut unter Verwendung von im Stand der Technik bekannten Verfahren isoliert werden und dann beispielsweise mittels Säulenchromatographie gereinigt werden. Monoklonale Antikörper können beispielsweise in Übereinstimmung mit dem bekannten Verfahren von Winter & Milstein (Winter, G. & Milstein, C., Nature, 349, Seite 293 - 299, (1991)) hergestellt werden.

Spezifische polyklonale Antikörperseren, die gegen den humanen LT-β-Rezeptor gerichtet sind, können unter Anwendung konventioneller Methoden hergestellt werden, indem Tiere, beispielsweise Ziegen, Kaninchen oder Mäuse, subkutan mit einem LT-β-Rezeptor abgeleitetem Protein oder Peptid oder -derivat, das das aktivierende Epitop repräsentiert, injiziert werden.
Das aktivierende natürliche Epitop kann dabei auch durch jedwede chemische Substanzen dargestellt werden, die eine dem natürlichen Epitop vergleichbare zu bindende Oberflächenstruktur und/oder Form und/oder Ladung aufweist.
Hierbei können auch die Immunreaktion verstärkende intraperitonale oder subkutane Injektionen von zusätzlichen Agentien (Adjuvantien, z.B. Freunds Adjuvanz) verwendet werden. Polyklonale Antiseren, die die gewünschten Antikörper enthalten, die gegen das aktivierende Epitop des LT-β-Rezeptor gerichtet sind, können durch intraperitonale sich wiederholende Immunisierung von Mäusen mit LT-β-Rezeptor abgeleitetem Protein oder Peptid oder -derivat in Abwesenheit vom Adjuvanz expandiert werden. Die Immunisierung der Tiere durch LT-β-Rezeptoren, abgeleiteten Proteinen, Peptiden oder -derivaten, welche das aktivierende Epitop repräsentieren, kann als Injektionen sowohl intraperitoneal als auch intravenös erfolgen.
Zur Herstellung von monoklonalen Antikörpern, können Hybridomazellen nach klassischen Methoden fusioniert werden und beispielsweise mittels ELISA (Ling et al., J. Interferon and Cytokine Res., 15, Seite 53 - 59 (1995)) gescreent werden. Hybridomazellen werden weiterhin auf ihre Fähigkeit, Antikörper zu produzieren, die den LT-β-Rezeptor, abgeleitetes Protein, Peptid, -derivat oder das aktivierende Epitop erkennen, und die Fettzelldifferenzierung zu modulieren, untersucht. Reine monoklonale Antikörper (IgG) wurden von Hybridomazellkulturüberständen mittels Protein A Sepharose aufgereinigt.

Zahlreiche Formen von anti-LT-β-R-Antikörpern können ebenso unter Anwendung von Standardmethoden zur Produktion von rekombinanter DNA hergestellt werden (Winter und Milstein, Nature, 349, Seite 293 - 299 (1991)). Zum Beispiel können "chimäre" Antikörper, in denen die Antigenbindestelle von einem Tierantikörper mit einer humanen konstanten Domäne verknüpft ist, hergestellt werden (z.B. Cabilly et al., US 4,816,567; Morrison et al., Proc. Natl. Acad. Sci. U.S.A., 81, Seite 6851 - 6855 (1984)). Chimäre Antikörper reduzieren die beobachtete Immunantwort, was in humanen klinischen Studien deutlich wird, in denen Tierantikörper verwendet werden. Entsprechend der vorliegenden Erfindung wird deshalb der Ausdruck Antikörper und Antikörperfragment auch dahingehend verstanden, dass Antikörper und/oder Antigenbindende Teile davon eingeschlossen sind, welche rekombinant hergestellt wurden, und, wenn erforderlich, modifiziert wurden, beispielsweise chimäre Antikörper, humanisierte Antikörper, multifunktionelle Antikörper, bispezifische oder oligospezifische Antikörper, Einzelstrangantikörper und F(ab)- oder F(ab)₂-Fragmente (siehe beispielsweise EP 368 684B1, US 4,816,567, US 4,816,397, WO 88/01649, WO 93/06213 oder WO 98/24884).

Zudem können rekombinante "humanisierte Antikörper", die das aktivierende Epitop des LT-β-Rezeptors erkennen, synthetisiert werden. Humanisierte Antikörper sind Chimären, die zum größten Teil humane IgG-Sequenzen aufweisen, in die die für die spezifische Antigenbindung verantwortliche Regionen inseriert wurden (WO94/04679). Tiere werden mit dem gewünschten Antigen immunisiert, die entsprechenden Antikörper isoliert und der Teil der variablen Sequenzregionen, die für die spezifische Antigenbindung verantwortlich sind, entfernt. Die von den Tieren stammenden Antigenbindestellen werden dann in die entsprechende Position der humanen Antikörpergene kloniert, in denen die humane Antigenbindestellen deletiert wurden. Humanisierte Antikörper reduzieren die Verwendung von heterologen (innerartlichen) Sequenzen in humanen Antikörpern und sind weniger riskant, eine Immunantwort in dem behandelten Individuum zu induzieren.

Die Herstellung unterschiedlicher Klassen rekombinanter anti-LT-β-R-Antikörpern, die das aktivierende Epitop erkennen, kann auch erzielt werden, indem chimäre oder humanisierte Antikörper mit anti-LT-β-R-variablen Domänen und humanen konstanten Domänen (CH1, CH2, CH3) hergestellt werden, die aus unterschiedlichen Klassen von Immunglobulinen isoliert wurden. Zum Beispiel können anti-LT-β-R-Antikörper, die das rezeptor-aktivierende Epitop erkennen, mit einer erhöhten Affinität für Antigenbindungsstellen rekombinant hergestelt werden, indem die Antigenbindungsstellen in Vektoren kloniert werden, welche korrespondierende humane konstante Regionen enthalten (Arulandam et al., J.Exp. Med., 177, Seite 1439 ― 1450 (1993); Lane et al., Eur. J. Immunol., 22, Seite 2573 ― 2578 (1993); Traunecker et al., Nature, 339, Seite 68 - 70 (1989))

Als Alternative zu klassischen Antikörpern ist es auch möglich, Proteingerüste (sogenannte "protein scaffolds") gegen das jeweilige Molekül zu verwenden, beispielsweise Anticaline, die auf Lipocalin basieren (Beste et al., Proc. Natl. Acad. Sci. USA, 96, Seite 1898-1903, (1999)). Die natürlichen Liganden-Bindungsstellen von Lipocalinen, beispielsweise von Retinol-bindenden Protein oder Bilin-bindenden Protein, können verändert werden, beispielsweise unter Verwendung eines "kombinatorischen Protein-Design"-Ansatzes, und zwar auf eine solche Weise, dass sie ausgewählte Haptene binden, (Skerra, Biochem. Biophys. Acta, 1482, Seite 337 ― 350, (2000)). Von anderen Proteingerüsten ist bekannt, dass sie Alternativen für Antikörper darstellen (Skerra, J. Mol. Recognit, 13, Seite 167-287, (2000)). Alle diese Protein-abgeleiteten Alternativen zu Antikörpern können als Bindeproteine bezeichnet werden. Entsprechend der vorliegenden Erfindung wird deshalb der Ausdruck Bindeprotein dahingehend verstanden, dass die hierin beschriebenen Bindeproteine und Bindemoleküle z.B. Affiline, Anticaline oder Aptamere ebenfalls eingeschlossen sind, welche spezifisch das rezeptor-aktivierende Epitop des LT-β-Rezeptors, daraus abgeleiteten Proteinen, Peptiden oder ―derivaten erkennen, welche rekombinant hergestellt und, wenn erfordertich, modifiziert wurden.

### Identifizierung von Epitopen des LT-β-R, welche bei Bindung durch anti-LT-β-R-Antikörper eine Rezeptoraktivierung zur Folge haben

Antikörper binden mit definierter Spezifität und Affinität an die dazugehörigen Zielmoleküle. So auch Antikörper, die gegen Rezeptoren auf der Zelloberfläche gerichtet sind. Die Bindung eines Antikörpers an einen solchen Oberflächenrezeptor, hier LT-β-R, kann dessen Aktivierung bewirken. Dazu ist die blosse Bindung des Antikörpers notwendig, aber nicht hinreichend. Vielmehr ist die Rezeptoraktivierung abhängig von der Art der Bindung des Antikörpers. Diese Art der Bindung durch den Antikörper muss den Effekt simulieren, welchen die Bindung eines natürlichen Liganden an den Rezeptor hat. In vielen Fällen bewirkt der natürliche Ligand eine Änderung der Form (Konformation) oder einer anderen Eigenschaft des Rezeptors, zum Beispiel seine Dimerisierung oder Multimerisierung, oder beides. Eine Aktivierung erfolgt dann, wenn diese strukturellen Änderungen eine intrinsische Aktivität des Rezeptorproteins, zum Beispiel eine Enzymaktivität, oder eine Protein-Proteinwechselwirkungsaktivität direkt oder indirekt zur Folge haben. Die induzierten Veränderungen des Rezeptors sind abhängig von der molekularen Position oder den Positionen auf dem Rezeptor, an den/die der Ligand oder Antikörper bindet. Molekulare Positionen, welche von Antikörpern erkannt werden, werden als Epitope bezeichnet. Dementsprechend binden Antikörper, welche zur Aktivierung von Rezeptoren führen, an solche Epitope, die bei Bindung die Rezeptoreffekte der Ligandenbindung (s.o.) simulieren.
Die Lage eines solchen Epitops im Antigen kann kartiert werden, wenn mindestens ein aktivierender Antikörper (auch ein agonistischer Antikörper genannt) zur Verfügung steht. Hierzu wird das Protein des Rezeptors, hier LT-β-R, zunächst in definierte Teilstücke zeriegt, was entweder durch rekombinante Produktion von Teilsequenzen des Rezeptors geschehen kann, oder auch durch Synthese von Teilpeptiden des Rezeptors. Solche Fragmente oder Teilpeptide des Rezeptors werden daraufhin analysiert, ob sie von agonistischen Antikörpern erkannt werden. Diese Analyse kann mittels etablierter Techniken, z.B. ELISA, durchgeführt werden, oder auch mittels neuerer Bindungstechniken von Antikörpern an Peptid-Arrays. Bei letzterem werden überlappende Teilpeptide des Rezeptors LT-β-R in Form von Arrays immobilisiert. Diese Arrays werden nach Vorbehandlung mit Blockierungspuffer mit einem agonistischen anti-LT-β-R-Antikörper versehen und für eine ausreichende, vom Antikörper abhängende Dauer inkubiert, danach gewaschen. Zuletzt wird der an spezifische Peptide gebundene agonistische Antikörper mit sekundärem Antikörpern auf Mikroarray-Scannem nachgewiesen. Die derartig nachweisbare Bindung an teilweise überlappende Peptide, welche aus dem LT-β-R abgeleitet wurden, ermöglicht eine genaue Kartierung und damit Definition der Epitope, welche bei Antikörper-Bindung zur Induktion der Rezeptor-Signaltransduktion führen. Diese Peptide können dann gezielt zur Herstellung von weiteren agonistischen anti-LT-β-R Antikörpern verwendet werden, welche sämtlich dadurch charakterisiert sind, dass diese das definierte Peptid erkennen. Durch Kombinationen und Verbindungen von mehreren Peptiden ist es ebenfalls möglich, agonistische anti-LT-β-R Antikörper zu generieren, die gegen diskontinuierliche Epitope gerichtet sind, d.h. solche Antikörper, die zwei oder mehrere in der Primärsequenz nicht verbundene Teilsequenzen gleichzeitig erkennen. Es ist ebenfalls möglich, artifizielle chemische Strukturen als Epitope zu verwenden, welche den beschriebenen natürlichen kontinuierlichen oder diskontinuierlichen Epitopen in Form, Ladung, Struktur oder anderen für die Epitopbindung relevanten Eigenschaften entsprechen, ohne dass die originale Peptidsequenz in den Strukturen selbst enthalten ist. Diese Peptide, Epitope oder artifizielle chemische Strukturen, welche den Epitopen funktionell entsprechen, können sowohl zur Immunisierung von Tieren zur Generierung beispielsweise polyklonaler oder monoklonaler Antikörper als auch zur Generierung von Antikörperderivaten und Bindeproteinen mittels rekombinanter Technologien verwendet werden. Zusätzlich ist es ebenfalls möglich, diese aktivierenden Epitope als Immunogen zur Bildung von aktivierenden Antikörpern direkt im Menschen zu verwenden. Dieses ermöglicht eine Vakzinierung von Patienten, mittels derer Adipositas und deren Folgeschäden behandelt werden kann.

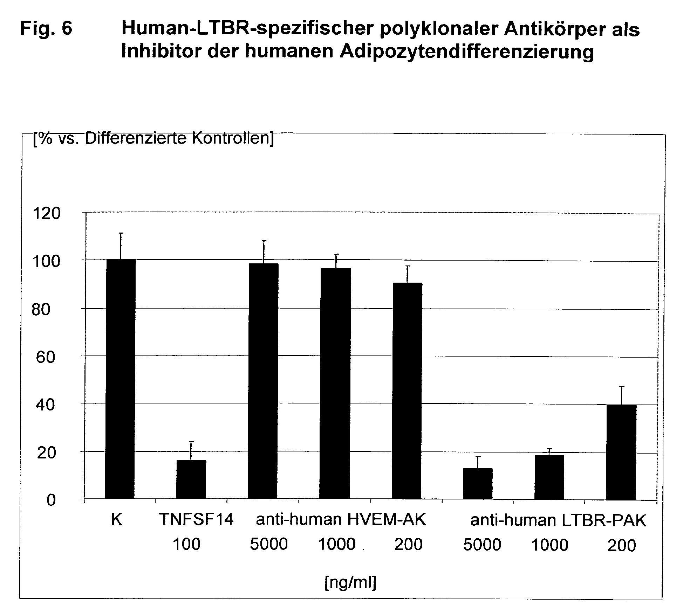

Die Identifizierung (mittels der oben beschriebenen Methoden) von Epitopen, welche bei Bindung zur Rezeptoraktivierung führen, dient unter anderem dazu, mit rationalen Verfahren gezielt agonistische Antikörper zu generieren. Andererseits ist es möglich, Informationen über Epitope, welche bei Bindung zur Rezeptoraktivierung führen, und insbesondere über Antikörper, welche solche Epitope binden, auch über Kompetitionsexperimente zu erlangen. So können schon vorhandene agonistische Antikörper, beispielsweise diejenigen, welche in Beispiel 2 und Figur 6 als agonistische anti-LT-β-R Antikörper dargestellt sind, zur Identifizierung und Charakterisierung von weiteren agonistischen Antikörpern verwendet werden. Bei einem solchen Antikörper handelt es sich beispielsweise um den agonistischen humanspezifischen polyklonalen Antikörper (Ziege IgG anti-human Lymphotoxin-beta-Rezeptor polyklonaler Antikörper, AF629, Fig. 6). Da die in Beispiel 2 und Figur 6 als agonistische anti-LT-β-R Antikörper beschriebenen Antikörper ein Epitop des LT-β-R erkennen, welches bei Bindung die Rezeptoraktivierung auslöst, werden solche Antikörper oder Antikörperpräparationen oder Formulierungen, die an dasselbe Epitop binden, ebenfalls den Rezeptor aktivieren. Kompetitionsexperimente, bei denen die zu analysierenden Antikörper daraufhin getestet werden, ob sie bei Zugabe im Überschuss die Bindung eines vorgegebenen (in diesem Fall beispielsweise des in Beispiel 2 und Figur 6 als agonistische anti-LT-β-R Antikörper beschriebenen) Antikörpers an den Rezeptor verhindern können (= kompetitieren können) können dazu verwendet werden, Antikörper zu finden, die an das gleiche Epitop binden wie ein verwendeter agonistischer anti-LT-β-R-Antikörper. Solche Kompetitionsanalysen, deren Durchführung von einem Fachmann mittels Standardmethoden durchführbar sind, können dementsprechend ebenfalls zur Analyse der potentiellen Bindung von neuen Antikörpern an aktivierende Epitope des LT-β-R verwendet werden.

### Modulation der Fettzelldifferenzierung durch spezifische Aktivierung der LT-β-R vermittelten Signaltransduktion

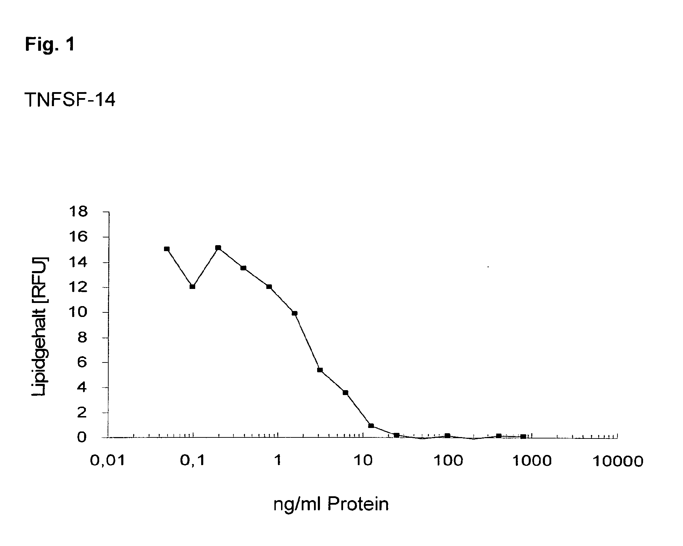

Gemäß einer bevorzugten Ausführungsform wirken die erfindungsgemäß verwendeten anti-LT-β-R-Antikörper, -Antikörperderivate oder -Bindeproteine dadurch, dass sie die Bildung terminal differenzierter Adipozyten hemmen. Dabei kann die Bildung terminal differenzierter Adipozyten zum einen dadurch gehemmt werden, dass die Differenzierung zu terminal differenzierten Adipozyten gehemmt wird, insbesondere durch Hemmung des Transkriptionssignalweges zur Differenzierung. Zum anderen kann die Bildung terminal differenzierter Adipozyten durch Hemmung der Lipogenese oder durch Förderung der Lipolyse gehemmt werden. Die Hemmung der Differenzierung zu terminal differenzierten Adipozyten kann durch Messung des Lipides (z.B. durch Färbung mit Nile Red) erfolgen. Mit Reportergenassays wie z.B. für PPAR-γ oder CEBP-β oder -δ lässt sich die Hemmung des Transkriptionssignalweges messen. Durch Färbung mit lipophilen Farbstoffen wie z.B. Nile Red lässt sich der Lipidgehalt in Adipozyten nachweisen. Die Modulation der Adipozytendifferenzierung durch Aktivierung von LT-β-R ist sowohl für die natürlichen Rezeptor-Liganden TNFSF14, LT-α1/β2 als auch für aktivierende anti-LT-β-R Antikörper in Beispiel 2 beschrieben. Hierbei wird sowohl in murinen Zellen als auch in frischen menschlichen Präadipozyten der Effekt durch die Liganden- (TNFSF14) und Antikörper (anti-LT-β-R) - vermittelte Signaltransduktion klar und in einer dosisabhängigen Weise darstellbar (siehe Figuren 1-3,5,6).

Weil eine Involvierung von LT-β-R-vermittelter Signaltransduktion in apoptotische Prozesse und Zelltod (beispielsweise in Tumorzellen) schon bekannt ist, wurde ebenfalls analysiert, ob die Hemmung der Differenzierung von Adipozyten mit Induktion von Apoptose oder Zelltod assoziiert ist. Die Bestimmung von Apoptose ist beispielsweise durch CDD+ Assay (Cell Death Detection ELISA PLUS, Roche Diagnostics GmbH, Mannheim, Deutschland), DiOC6-Assay, AnnexinV-Assay oder CaspaTag™-Assay (CaspaTag™Caspase-3 (DEVD) Activity Kit, Intergen Company, Purchase, NY, USA) möglich. Die Bestimmung von Lebendzellzahlen ist beispielsweise durch 'Alamar Blue' oder 'CellTiterGlow' Assays möglich.

Die Möglichkeit von Toxizität oder Induktion von Apoptose durch Aktivierung von LT-β-R wurde von der Anmelderin in murinen und menschlichen Zellen untersucht und ist in Beispiel 3 beschrieben. Es konnte gezeigt werden, dass unter den verwendeten Bedingungen keine Toxizität auftrat. In diesen Experimenten wurde ebenfalls demonstriert, dass der hemmende Effekt reversibel ist. Dieses konnte durch Wegnahme des Proteins gezeigt werden, was in Folge den Zellen einen Wiedereinstieg in den Differenzierungsprozess ermöglicht. Offensichtlich ist dieses nur lebenden Zellen möglich, was auch durch mikroskopische Messungen der lebendigen Zellen bestätigt werden konnte.

Therapeutische Verwendung von Antikörpern und anderen Aktivatoren der LT-β-R vermittelten Signaltransduktion zur Behandlung von Adipositas und assoziierten Krankheiten

Die Erfindung betrifft ein Verfahren zur Behandlung eines Patienten, bei welchem dem Patienten eine wirksame Menge eines und/oder mehrerer der oben definierten agonistischen Agenzien, wie anti-LT-β-R Antikörper, verabreicht wird. Bevorzugt leidet der Patient unter Adipositas. Für dieses erfindungsgemäße Verfahren gelten alle oben für die erfindungsgemäße Verwendung aufgeführten Ausführungsformen.

Wie bereits oben beschrieben, ermöglichen die erfindungsgemäß gefundenen Faktoren TNFSF14 und anti-LT-β-R Antikörper neben der Inhibition der Differenzierung von Präadipozyten zu Adipozyten eine Hemmung der Funktion der reifen bzw. des reifenden Adipozyten durch die Hemmung der Lipogenese.

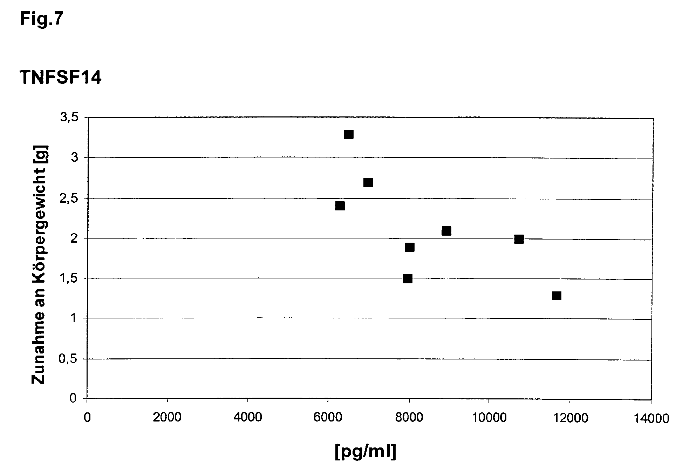

Die Beobachtungen, dass gezielte Rezeptoraktivierung von LT-β-R durch rekombinante Supplementation des rezeptoraktivierenden Liganden TNFSF14 das Gewicht von Tieren reduzieren kann (s. Beispiel 4, Figur 7), ist ein Beweis für das hier beschriebene therapeutische Konzept.

Unter Inhibieren bzw. Hemmen wird erfindungsgemäss das vollständige oder teilweise Unterdrücken einer biologischen Funktion verstanden. In unserem speziellen Fall ist die zu inhibierende Funktion die Differenzierung von Adipozyten in adipösen Patienten. Entsprechend der vorliegenden Erfindung beschreibt der Ausdruck "Inhibitor" ein Agenz, wie einen anti-LT-β-R-Antikörper, das vorzugsweise den Prozess der Fetteinlagerung selbst inhibiert. In diesem Fall kann der aktivierende Antikörper als Inhibitor bezeichnet werden. Die Inhibition kann beispielsweise durch Induktion von LT-β-R -abhängiger Signaltransduktion und darauffolgende Modulation der Expression regulierter spezifischer Gene erreicht werden.

Gemäß einer bevorzugten Ausführungsform wirkt der verwendete Inhibitor, z.B. der anti-LT-β-R-Antikörper, dadurch, dass er die die Bildung terminal differenzierter Adipozyten hemmt. Dies kann beispielsweise und präferentiell geschehen durch Interferenz der Differenzierung zu terminal differenzierten Adipozyten, insbesondere durch Modulation des LT-β-R-Transkriptionssignalweges bei der Differenzierung in Präadipozyten.

Die Erfindung betrifft ferner eine pharmazeutische Zusammensetzung, welche ein oder mehrere Agenzien der vorliegenden Erfindung enthält. Für alle pharmazeutischen Zusammensetzungen gemäß der vorliegenden Erfindung gilt das folgende:

Für die Herstellung der erfindungsgemäßen pharmazeutischen Zusammensetzung werden die entsprechenden Moleküle gewöhnlich mit geeigneten Additiva oder Hilfsstoffen formuliert, wie beispielsweise physiologische Pufferlösung, beispielsweise Natriumchloridlösung, demineralisiertes Wasser, Stabilisatoren, wie Protease- oder Nukleaseinhibitoren, vorzugsweise Aprotinin, ε-Aminocapronsäure oder Pepstatin A oder Chelatbildner wie EDTA, Gelformulierungen, wie weiße Vaseline, Paraffin mit geringer Viskosität oder Gelbwachs (yellow wax), abhängig von der Art der Verabreichung.

Geeignete zusätzliche Additiva sind beispielsweise Detergenzien, wie beispielsweise Triton X-100 oder Natriumdeoxycholat, aber auch Polyole, wie beispielsweise Polyethylenglycol oder Glycerin, Zucker, wie beispielsweise Sucrose oder Glucose, zwitterionische Verbindungen, wie beispielsweise Aminosäuren, wie Glycin oder insbesondere Taurin oder Betain oder ein Protein, wie beispielsweise bovines oder humanes Serumalbumin. Detergenzien, Polyole und/oder zwitterionische Verbindungen sind bevorzugt.

Die physiologische Pufferlösung hat vorzugsweise einen pH von etwa 6,0-8,0, insbesondere einen pH von etwa 6,8-7,8, insbesondere einen pH von etwa 7,4, und/oder eine Osmolarität von ungefähr 200-400 milliosmol/Liter, vorzugsweise von etwa 290-310 milliosmol/Liter. Der pH des Medikaments wird im Allgemeinen unter Verwendung eines geeigneten organischen oder anorganischen Puffers angepasst, wie beispielsweise unter Verwendung von Phosphatpuffern, Trispuffern (Tris(hydroxymethyl)aminomethan), HEPES-Puffer ([4-(2-Hydroxyethyl)piperazino]-ethansulfonsäure) oder MOPS-Puffer (3-Morpholino-1-propansulfonsäure). Die Wahl des entsprechenden Puffers hängt im Allgemeinen von der gewünschten Puffermolarität ab. Phosphatpuffer ist beispielsweise geeignet für Injektions- oder Infusionslösungen.

Injektionslösungen werden im allgemeinen verwendet, wenn nur eine geringe Menge einer Lösung oder Suspension, beispielsweise etwa 1 bis etwa 20 ml, verabreicht werden sollen. Infusionslösungen werden im allgemeinen verwendet, wenn eine größere Menge einer Lösung oder Suspension, beispielsweise ein oder mehrere Liter, verabreicht werden sollen. Da im Gegensatz zur Infusionslösung nur wenige Milliliter im Fall von Injektionslösungen verabreicht werden, sind geringe pH-Differenzen oder des osmotischen Drucks des Bluts oder der Gewebeflüssigkeit im Vergleich zur Injektionslösung selbst nicht bemerkbar oder spielen keine große Rolle. Die Verdünnung der Formulierung vor der Verwendung ist daher im allgemeinen nicht erforderlich. Wenn allerdings relativ große Mengen verabreicht werden, sollte die erfindungsgemäße Formulierung kurz vor der Verabreichung verdünnt werden, so dass etwa eine isotonische Lösung erhalten wird. Ein Beispiel einer isotonischen Lösung ist eine 0,9%-ige Kochsalzlösung.

Grundsätzlich wird die wirksame Dosis vom Gewicht und dem Zustand des jeweiligen zu behandelnden Subjektes abhängen. Dabei ist davon auszugehen, dass dem Fachmann bekannt ist, wie eine geeignete Dosierung bestimmt werden kann.

Die pharmazeutische Zusammensetzung kann auf verschiedene Art und Weise verabreicht werden, beispielsweise intramuskulär, subkutan, intrathekal, in das Fettgewebe, perkutan (gelöst in DMSO), intravenös oder intraperitoneal, oder durch Fusion oder Gele, welche das jeweilige Medikament enthalten. Es ist femer möglich, das Medikament topisch und lokal zu applizieren. Ferner kann das Medikament durch ein transdermales therapeutisches System (TTS) verabreicht werden, welches eine zeitlich kontrollierte Abgabe des Medikaments ermöglicht. TTS sind beispielsweise aus EP 944398, EP 916366, EP 889723 oder EP 852493 bekannt. Eine langsamere Abgabe des Proteins, Peptids, oder Antikörpers wird durch Kombination mit Polymeren, eine längere Halbwertszeit durch Zugabe von PEG erreicht. Geeignete Polymere erlauben auch eine perorale Verabreichung der pharmazeutischen Zusammensetzung, beispielsweise durch eine geschützte Passage durch den Darm und die gezielte Penetration der Zell-Zell-Kontaktstellen im Bereich des Darmepithels. Eine Kombination mit chemischen Wirkstoffen (z.B. Appetithemmer, Lipasehemmer) kann die Wirkung noch verstärken.

Gemäß einer bevorzugten Ausführungsform liegt die pharmazeutische Zusammensetzung mit einem aktivierenden Agenz gemäß der vorliegenden Erfindung in einer Form vor, welche den Kontakt des Wirkstoffes mit einem Bestandteil der Zelloberfläche (nicht zellgängig) ermöglicht (Cardiovasc Pharmacol Ther., 7 (3) Seite 171 ― 80, (Juli 2002)).

Die Aufzählungen in Gestalt der oben angeführten Erwähnungen sollen die vorliegende Erfindung verdeutlichen, jedoch nicht limitieren.

Die Erfindung betrifft femer ein Verfahren zur Behandlung eines Patienten, bei dem Patienten eine wirksame Menge eines oder mehrerer der oben definierten Inhibitoren verabreicht wird. Bevorzugt leidet der Patient unter Adipositas oder Diabetes Typ II-oder unter Diabetes Typ II-verwandten Krankheiten.

### Beispiele

Die Erfindung wird im folgenden durch Beispiele und Figuren erläutert, die in keiner Weise dazu gedacht sind, den Gegenstand der Erfindung einzuschränken, sondern sie stellen Ausführungsformen dar.

In den Beispielen wird insbesondere gezeigt, dass die erfindungsgemäß verwendeten löslichen Polypeptide und Antikörper in der Lage sind, die Lipideinlagerung in murinen 3T3-L1 Zellen und in menschlichen Präadipozyten zu inhibieren. Die Inhibition konnte sowohl in primären humanen Adipozyten subkutanen als auch omentalen Ursprungs gezeigt werden. Der Nachweis erfolgte jeweils durch Nile Red-Assay.

Femer wird gezeigt, dass der Rezeptor LT-β-R, dessen Aktivierung die Modulation der Fettzelldifferenzierung bewirkt, auf menschlichen und murinen Fettzellvorläuferzellen vorhanden ist. Diese Tatsache ist eine wichtige Voraussetzung, um den beobachteten Phänotyp durch rezeptorvermittelte Signaltransduktion zu erklären.

Desweiteren wird gezeigt, dass die Aktivierung des Rezeptors LT-β-R, welche die Modulation der Fettzelldifferenzierung bewirkt, in unserem System muriner und humaner Zellkulturen zu keinen toxischen Effekten führt.

Zusätzlich wird gezeigt, dass exprimierte lösliche murine homologe Varianten von TNFSF14 bzw. dessen Proteinfragmente in der Lage sind, die Gewichtszunahme in Mäusen zu reduzieren, ohne generelle toxische Effekte zu verursachen.

Es wird ebenfalls gezeigt, dass Antikörper, welche durch Rezeptoraktivierung den Fettzell-Modulierenden Phänotyp auslösen, definierte Epitope des LT-β-Rezeptors binden und dadurch die Signaltransduktion auslösen. Diese Tatsache ist eine wesentliche Voraussetzung für rationale Generierung von definierten Antikörpern, welche durch gezielte Bindung an diese Epitope des Rezeptors die Signaltransduktion aktivieren.

### Beschreibung der Figuren:

Fig. 1:
   Dargestellt ist die Dosis Wirkungskurve, die den Lipidgehalt von 3T3-L1 Zellen in Abhängigkeit der zugesetzten Menge an rekombinantem löslichem bzw. sekretiertem bzw. löslichem Proteinfragment TNFSF-14 zeigt.
   Die Messergebnisse sind als relative Fluoreszenzeinheiten (RFU) am Tag 5 nach Zugabe des Proteins angegeben.
Fig. 2:
   Dargestellt ist die prozentuale Hemmung der Lipideinlagerung am Tag 8 in Form einer Dosis-Wirkungskurve durch jeweils rekombinante sekretierte bzw. lösliche Proteinfragmente von TNFSF14 in primären humanen Adipozytenzellkulturen subkutanen Ursprungs. Dazu wurden die primären humanen Zellkulturen mit den jeweiligen rekombinanten Proteinen inkubiert. Die prozentualen Daten beziehen sich jeweils auf Kontrollkulturen, die mit einer noch unwirksamen Proteinkonzentration von 0,025ng/ml behandelt wurden. Eine niedrige Signalstärke bedeutet geringe Lipideinlagerung in die differenzierenden humanen Adipozyten, eine hohe Signalstärke bedeutet eine starke Lipideinlagerung.
Fig. 3:
   Dargestellt ist die Hemmung der Lipideinlagerung durch zugesetzte rekombinante lösliche Proteinfragmente von alpha1/beta2-Lymphotoxin, alpha2/beta1-Lymphotoxin im Vergleich zu TNFSF14 in primäre humane Adipozyten subkutanen Ursprungs. Die Messergebnisse sind als relative Fluoreszenzeinheiten (RFU) am Tag 8 nach erstmaliger Zugabe des jeweiligen Proteins angegeben.Eine niedrige Signalstärke bedeutet geringe Lipideinlagerung in die differenzierenden humanen Adipozyten, eine hohe Signalstärke bedeutet eine starke Lipideinlagerung.

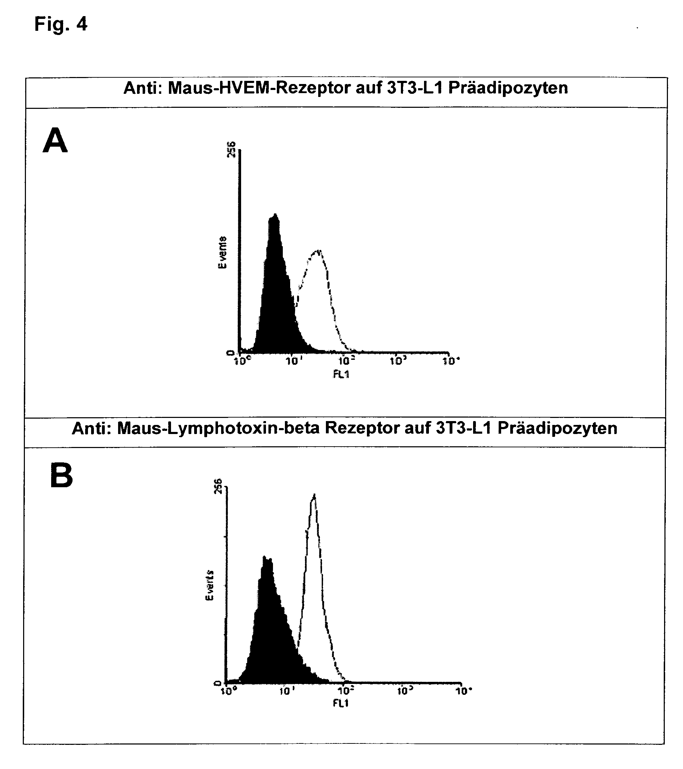

Fig. 4: Rezeptornachweis mHVEM-mLT-β-R
   Dargestellt sind die Nachweise der in der Literatur beschriebenen Rezeptoren von TNFSF14 ((A) HVEM-Rezeptor und (B) Lymphotoxin-beta Rezeptor mittels spezifischer Rezeptor-Antikörper (Ziege IgG anti-Maus HVEM polyklonaler Antikörper R&D Systems, Wiesbaden/Deutschland Kat.No. AF2516 und Ratte IgG2a anti-Maus-Lymphotoxin-beta-Rezeptor monoklonaler Antikörper, Klon 4H8WH2; Immunogen Aminosäuren 31-221, dies entspricht der gesamten extrazellulären Domäne des reifen Maus Rezeptorproteins; Fa. Axxora, Deutschland Kat.No. ALX ALX-804-145) und geeigneter Fluorophor markierter sekundärer Antikörper und dem Fluoreszenzaktivierten Zellsortierungsverfahren (FACS) auf 3T3-L1 Zellen. Die Messergebnisse sind als Fluoreszenzintensitäten (FL-1) im Verhältnis zur Ereigniszahl ("event") angegeben. Verglichen werden jeweils das Fluoreszenz-Intensitätsprofil in Anwesenheit (□) bzw. Abwesenheit (■) des spezifischen Rezeptor-Antikörpers. Eine Verschiebung der Fluoreszenzintensität hin zu stärkeren Intensitäten (Rechtsverschiebung), bedeutet eine Bindung des spezifischen Rezeptorantikörpers gepaart mit dem Fluoreszenz-markierten sekundären Antikörpers an die Zelloberfläche. Dies zeigt die Rezeptor-Exposition an der Zelloberfläche an.
Fig. 5:
   Dargestellt sind die Dosis-Wirkungskurven eines agonistisch wirkenden Antikörpers für den Lymphotoxin-beta-Rezeptor (Ratte IgG2a anti-Maus-Lymphotoxin-beta-Rezeptor monoklonaler Antikörper, Klon 4H8WH2; Immunogen Aminosäuren 31-221, dies entspricht der gesamten extrazellulären Domäne des reifen Maus Rezeptorproteins; Fa. Axxora, Deutschland Kat.No. ALX ALX-804-145), und von TNFSF14, sowie zwei weiteren bekannten alternativen Liganden für den Lymphotoxin-beta Rezeptor- Lymphotoxin a1b2 und Lymphotoxin a2b1. Der Lipidgehalt von 3T3-L1 Zellen wurde in Abhängigkeit der zugesetzten Menge an rekombinantem sekretiertem bzw. löslichem Proteinfragment TNFSF14, alpha1/beta2-Lymphotoxin, alpha2/beta1-Lymphotoxin bzw. dem mausspezifischen monoklonalen Antikörper gemessen. Die Messergebnisse sind als relative Fluoreszenzeinheiten (RFU) am Tag 5 nach Zugabe des jeweiligen Proteins angegeben. Eine niedrige Signalstärke bedeutet geringe Lipideinlagerung in die differenzierenden humanen Adipozyten, eine hohe Signalstärke bedeutet eine starke Lipideinlagerung.
Fig. 6:
   Dargestellt ist die konzentrationsabhängie Hemmung der Lipideinlagerung durch zugesetzte Antikörper (Ziege IgG anti-human Lymphotoxin-beta-Rezeptor polyklonaler Antikörper, Immunogen: extrazelluläre Domäne des humanen Lymphotoxin-beta-Rezeptors; Fa. R&D Systems, Deutschland Kat.No. AF629) im Vergleich zu TNFSF14 in primären humanen Adipozyten subkutanen Ursprungs. Gezeigt ist ebenfalls die ausbleibende Hemmung der Lipideinlagerung bei Verwendung eines HVEM spezifischen Antikörpers (Ratte IgG2a anti-human HVEM monoklonaler Antikörper, Klon Laury-1, Immunogen: Extrazelluläre Domäne von HVEM Aminosäuren 39-200, Fa. Axxora, Grünberg Deutschland Kat.No.: ALX-804-814). Die Messergebnisse sind in Prozent versus Lipideinlagerung von normal differenzierten Zellen angegeben. Die Daten wurden am Tag 8 nach erstmaliger Zugabe des jeweiligen Proteins und der Induktion zur Differenzierung erhoben. Eine niedrige Signalstärke bedeutet geringe Lipideinlagerung in die differenzierenden humanen Adipozyten, eine hohe Signalstärke bedeutet eine starke Lipideinlagerung.
Fig 7:
   Dargestellt ist die Restriktion der Körpergewichtszunahme in männlichen C57BU6 Mäusen, 2 Wochen nach Injektion eines geeigneten Expressionsvektors, in Abhängigkeit vom Ausmaß der Expression löslichen bzw. sekretierten Proteinfragments von murinem TNFSF14. Das Ausmaß der Expression der Proteinfragmente wurde mittels ELISA-Bestimmung an Serumproben bestimmt. Gezeigt ist die negative Korrelation von Proteinexpression und Körpergewichtszunahme.
Fig. 8:
   Dargestellt ist die insulinabhängige Glukoseaufnahme in primären humanen Adipozyten subkutanen Ursprungs. Unwirksame (10-10M) und wirksame (10-7 M) Konzentrationen an Insulin wurden eingesetzt. Wirksame Insulin-Konzentrationen bewirken eine gesteigerte zelluläre Glukoseaufnahme unter den gewählten Bedingungen (Kontrolle). Die Auswirkungen der Ko-Inkubation mit zugesetzten Protein TNFSF14 (100 ng/ml) sind gezeigt. TNF-alpha (50 ng/ml) diente in diesem Zusammenhang als Positivkontrolle, da seine Interferenz mit der insulinabhängigen Glukoseaufnahme in Adipozyten bereits beschrieben wurde. Die Messergebnisse sind angegeben als "insulinabhängige Aufnahme von pmol Glucose innerhalb von 20 Minuten".
Fig. 9:
   Dargestellt sind Untersuchungen zur proliferativen Aktivität bzw. Zytotoxizität von TNFSF14, auf subkonfluenten 3T3-L1 Zellkulturen. Die proliferative Aktivität bzw. Zellzahl wurde durch eine Lebend-Zellzahlbestimmung mittels des AlamarBlue Assays bestimmt und die Messergebnisse sind in relativen Fluoreszenzeinheiten (RFU) angegeben.

### Beispiel 1:

### Kultivierung von Präadipozytenzellen, ihre Induktion zur Differenzierung und die Messung intrazellulär eingelagerten Lipids.

### Für die Propagation und Analyse von murinen 3T3-L1 Zellen galt:

3T3-L1 Zellen sind Maus-Fibroblasten Zellen embryonalen Ursprungs, die unter geeigneten Bedingungen eine terminale Differenzierung zu reifen Fettzellen (Adipozyten) durchführen können. Deshalb werden die in Proliferationskultur gehaltenen 3T3-L1 auch als Präadipozyten bezeichnet. Sie wachsen in Mono-Layern und besitzen in etwa eine Verdopplungszeit von 24 h. Die hier verwendeten 3T3-L1 Zellen stammten von der ATCC (CL-173). Da die 3T3-L1 Zellen bei Konfluenz spontan differenzieren und dabei die Eigenschaften der Kultur verändert werden, wurden die Zellen nicht bis zur Konfluenz kultiviert (max. 80%).

Die Passagierung erfolgte in 2 Tagesabständen wobei dazwischen kein Mediumwechsel durchgeführt wurde. Die Zellen wurden erst ab Passage 5 und nur bis Passage 18 zum Screening verwendet. Die Erhaltungs-Kultur der 3T3-L1 Zellen erfolgte in T-75-Kulturflaschen. Zur Passagierung alle 2 Tage wurden diese mit 4,5 x 10⁵ Zellen / T-75-Flasche angeimpft. Es erfolgte kein Mediumwechsel bis zur nächsten Passage.

Die Aussaat der 3T3-L1 Zellen für das Experiment erfolgte in 96 well Mikrotiterplatten: Die 3T3-L1 Zellen wurden in einer Zelldichte von 1,5x10⁴Zellen/well in 200 µl Wachstumsmedium/well ausgesät. Die Zellen wurden dann ohne Mediumwechsel für 3 Tage inkubiert und anschließend zur Differenzierung wie weiter unten beschrieben induziert und rekombinantem löslichem bzw. sekretiertem Proteinfragment bzw. Antikörper in Kontakt gebracht. Das 3T3-L1 Wachstumsmedium (GM) besaß folgende Zusammensetzung: DMEM, 10%FCS, 1% Penicillin-Streptomycin, 2% Glutamin, 1% Na-Pyruvat). Das Differenzierungsmedium (DM) wurde grundsätzlich als 2fach-Konzentrat direkt nach Zugabe von rekombinantem Protein oder Antikörper zugegeben. Es besteht in dieser Form aus einem durch Induktionsfaktoren (200 nM Insulin, 1 mM IBMX und 2 µM Dexamethason) ergänzten 3T3-L1 Wachstumsmedium.

### Für die Isolierung von Adipozyten aus Fettgewebe und Kultivierung von primären humanen Präadipozyten galt:

Isolierung und Differenzierung von Präadipozyten erfolgte wie bei Hauner et al. beschrieben [Methods Mol Biol. 2001; 155: 239-47]. Das bei Operationen gewonnene Fettgewebe wird mechanisch von Bindegewebs- und Blutgefäßresten befreit und danach zerkleinert. Es folgte der Verdau mit 200 U/ml Collagenase NB4 (Serva Electrophoresis GmbH, Heidelberg) für 90 min bei 37 °C und 80 rpm in PBS mit 2 % BSA (3 ml Collagenaselösung/g Fettgwebe). Im Anschluss wurde für 10 Minuten bei 200 g zentrifugiert. Das entstandene Pellet enthielt neben den Präadipozyten auch Erythrozyten und Bindegewebsreste und wurde in Erythrozytenlysepuffer (155 mM NH₄Cl, 5.7 mM K₂HPO₄, 0.1 mM EDTA, pH 7.3) aufgenommen. Nach einer maximalen Inkubationszeit von 10 Minuten bei Raumtemperatur wurde über einen 150 µM Filter filtriert und wie oben beschrieben zentrifugiert. Da das verbleibende Pellet noch Bindegwebsreste enthielt, wurde nach Aufnahme in Präadiopozyten-Medium (DMEM/F12 (Invitrogen GmbH, Karlsruhe) supplementiert mit 8 mg/l Biotin, 4 mg/l Pantothenat, 1.79 g/l NaHCO₃ und 55 mg/l Pyruvat) erneut über eine 70 µM Filter gereinigt. Die Zellen wurden gezählt, erneut zentrifugiert und in DMEM/F12 mit 10 % FKS und 50 µg/ml Gentamycin resuspendiert. Die Aussaat erfolgte in einer Dichte von 40 000 bis 55 000 Zellen/cm². Die Zellen wurden bei 37 °C, 5 % CO₂ inkubiert.

### Induktion zur Differenzierung und Zugabe des zu testenden Überstandes bzw. des zu testenden Proteins auf 3T3L1 Zellen:

Drei Tage nach der Aussaat der 3T3-L1 Zellen wurden 140 µl Medium von den 3T3-L1 Zellen abgenommen und 50 µl Wachstumsmedium zugegeben, in dem geeignete Mengen an rekombinantem Protein (0-3 µg/ml Protein) bzw. Antikörper (zum Beispiel 0-10 µg/ml) gelöst wurden. Zusätzlich erfolgte nun die Zugabe von 100 µl 2X Differenzierungsmedium auf die 3T3-L1 Zellen gefolgt von einer Inkubation der 3T3-L1 Zellen im Brutschrank für 5 Tage. Nach dieser Zeit wurde eine Bestimmung des intrazellulär eingelagerten Lipids vorgenommen.

### Differenzierung von humanen Präadipozyten und SGBS Zellen:

Humane Präadipozyten wurden in einer Dichte von 40 000 bis 55 000 Zellen/cm² ausgesät. Für SGBS Zellen (beschrieben in Wabitsch et al., 2001, Int J Obes Relat Metab Disord. 25:8-15) lag die Zelldichte um den Faktor 10 niedriger. Nach der Aussaat verblieben die Zellen der Primärkultur für 20 Stunden, die SGBS Zellen für 3 Tage im Proliferationsmedium (Präadipozyten-Medium mit 10 % FKS und 50 µg/ml Gentamycin). Zur Induktion der Differenzierung wurden die Zellen zweimal mit PBS gewaschen und dann in serum-freiem Differenzierungsmedium inkubiert (Präadipozyten-Medium mit 66 nM Insulin, 1nM T₃, 100 nM Hydrocortison, 10 µg/ml Transferrin, 50 µg/ml Gentamycin). Für die ersten drei Tage wurden außerdem 1 µg/ml Troglitazon und 0,5 mM IBMX zugegeben. Nach dieser Zeit und im weiteren Verlauf alle drei bis vier Tage erfolgte ein Mediumwechsel mit Differenzierungsmedium. Die Zellen wurden verwendet, wenn mindestens 50% der Zellen (bezogen auf die Zellzahl) Fett eingelagert hatten.

### Messung des intrazellulär eingelagerten Lipids:

Zur quantitativen Bestimmung der Einlagerung von zellulärem Lipid wurde Nile Red-Reagenz (Molecular Probes, Leiden, Niederlande; CAS Nummer 7385-67-3) verwendet (Nile Red-Färbelösung: 4 µg/ml Nile Red in PBS / 40 % DMSO). Die Fluoreszenz wurde bei einer Exzitations-Wellenlänge von 485nm und einer Emissions-Wellenlänge von 590 nm gemessen. Die erforderliche Menge Nile Red wurde zur berechneten Menge DMSO zugegeben und gemischt. Anschließend wird die berechnete Menge PBS zugegeben und die Lösung gemischt. Zur Durchführung des Nile Red-Assay wurde am Tag 5 (3T3-L1 Zellen) bzw. Tag 8-12 (primäre humane Adipozytenkulturen) nach der Induktion zur Differenzierung wurde 140 µl Medium von den 3T3-L1 Zellkulturen abgenommen und 200 µl PBS zugegeben. Danach wurde 150 µl Flüssigkeit abgenommen und je 50 µl Nile Red-Färbe-Lösung zugegeben. Die Inkubation der Platten erfolgte für 4h bei 37°C im CO₂-Inkubator. Das Auslesen erfolgte in einem Fluoreszenz-Reader bei einer Exzitation von 485nm und einer Emission von 590 nm (200 msec Lesezeit). Die Messergebnisse sind als relative Fluoreszenzeinheiten (RFU) oder prozentual im Vergleich zur normal differenzierten (nicht inhibierten) Kontrolle angegeben.

### Beispiel 2:

### Inhibition der Lipideinlagerung in Zellkulturen mittels Aktivierung des Rezeptors durch rezeptorspezifische Antikörper bzw. naturgemässe Liganden.

Die Aktivierung eines zellulären Rezeptors kann durch den naturgemässen Liganden, als auch durch einen Antikörper bewerkstelligt werden, wenn die Aktivierung desselben zum Beispiel durch eine erfolgreiche Klusterbildung von Rezeptormolekülen wie im vorliegenden Fall bewirkt wird. Der Antikörper wirkt agonistisch, wenn er nach Bindung an seine Zielstruktur zu einer vergleichbaren Aktivität führt, wie sie der naturgemässe Ligand erzeugt. Dabei kann die Affinität abweichend sein. Das Epitop des Antikörpers kann, muss aber nicht zwingend der Bindungsstelle für den Liganden entsprechen. Entscheidend ist die funktionelle Wirkung auf den Aktivierungszustand des Rezeptors. Nicht jeder beliebige bindende Antikörper wird also eine Aktivierung des Rezeptors zur Folge haben, sondern nur solche, die an bestimmte Epitope in den extrazellulären Domänen des Rezeptors binden, die eine solche Aktivierung zur Folge haben.

Zunächst wurde ein Rezeptor durch seinen naturgemässen Liganden aktiviert. Dabei war im vorliegenden Fall der humane Ligand in der Lage auch den entsprechenden homologen Maus-Rezeptor zu aktivieren.

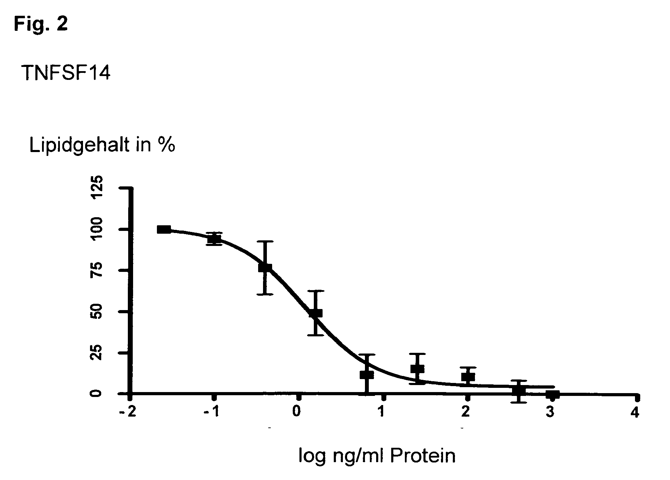

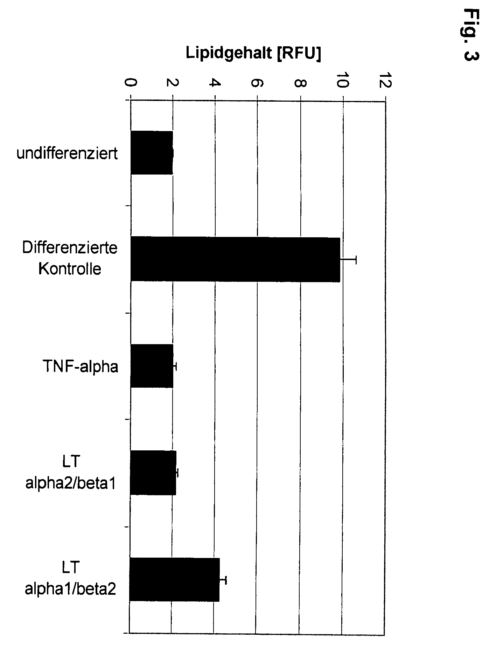

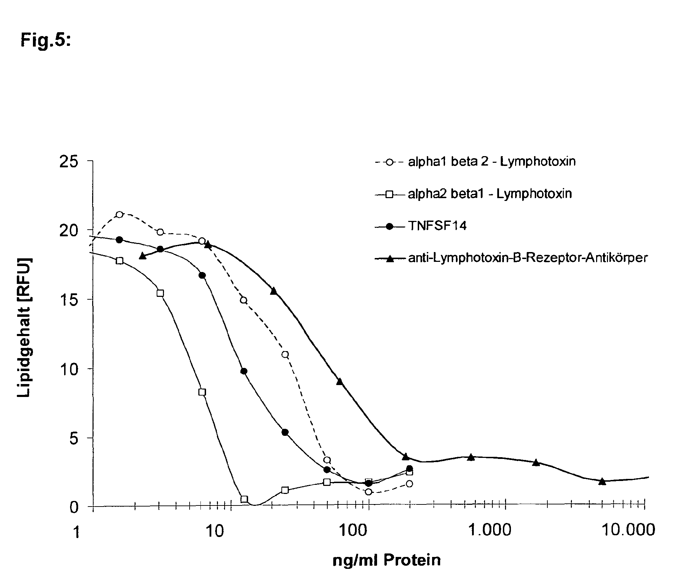

Die Hemmung der Lipideinlagerung in Maus 3T3-L1 Zellen (Fig.1) bzw. in primären humanen Adipozyten (Fig.2) wurde mit einem rekombinanten, sekretierten bzw. löslichen Proteinfragment von humanem TNFSF14 (R&D Systems GmbH, Wiesbaden: CatNo.: 664-LI [CD33 signal (met1-17)/10x His/GGGSGGGSGGGSIEGR/]-Asp-74 - Val-240) bestimmt. Die Ergebnisse zeigen, daß die Lipideinlagerung von murinen 3T3-L1 Zellkulturen (Fig.1) sowie in primären humanen Adipozyten (Fig.2) während der Differenzierung durch die Zugabe von rekombinant löslichem Proteinfragment von TNFSF14 dosisabhängig inhibiert werden kann. Die Lipideinlagerung kann als Maß für die Differenzierung der Adipozyten dienen, bzw. als ein Maß für die Regulation der Lipogenese oder Lipolyse in diesen Zellen. Es ergab sich in beiden zellulären Systemen eine halbmaximale Wirkkonzentration von etwa 2 ng/ml für das rekombinante lösliche Proteinfragment von TNFSF14. Eine ebenfalls derart vergleichbare Inhibition der Differenzierung wurde auch auf primären Fettzellen omentalen Ursprungs und der humanen SGBS Adipozytenzelllinie erreicht. Auch die alternativen Liganden humanes Lymphotoxin alpha1/beta2 und für Lymphotoxin alpha2/beta1 (je 100 ng/ml) für den Lymphotoxin-beta Rezeptor zeigten in 3T3-L1 Zellen (Fig.5) bzw. in primären humanen Adipozyten (Fig. 3) eine Hemmung der Lipideinlagerung.

Auf murinen 3T3-L1 Zellen wurde der agonistische mausspezifische monoklonale Antikörper (Ratte IgG2a anti-Maus-Lymphotoxin-beta-Rezeptor monoklonaler Antikörper, Klon 4H8WH2, Fig.5) gegen den Lymphotoxin-beta Rezeptor (LT-β-R) von TNFSF14 eingesetzt. Auf humanen Adipozyten fand der agonistische humanspezifische polyklonale Antikörper (Ziege IgG anti-human Lymphotoxin-beta-Rezeptor polyklonaler Antikörper, AF629, Fig. 6) Verwendung. Die Ergebnisse zeigen, dass die Lipideinlagerung in murinen 3T3-L1 Zellen sowie in primären humanen Adipozyten (Fig.6) während der Differenzierung durch die Zugabe von agonistischem Antikörper (4H8WH2 bzw. AF629) jeweils dosisabhängig inhibiert werden kann. Die Lipideinlagerung kann als Maß für die Differenzierung der Adipozyten dienen, bzw. als ein Maß für die Regulation der Lipogenese oder Lipolyse in diesen Zellen. Die Zugabe von 1 µg/ml humanspezifischen Rezeptorantikörper führte zu einer Inhibition der Differenzierung, die der vergleichbar war, wenn 100 ng/ml naturgemässer Ligand (TNFSF14) eingesetzt wurden (Fig.6). Die Zugabe von 50 ng/ml mausspezifischem Rezeptorantikörper Antikörper führte in 3T3-L1 Zellkulturen zu einer Inhibition der Differenzierung, die der vergleichbar war, wenn 15 ng/ml naturgemässer Ligand (TNFSF14) eingesetzt wurden (Fig.6). Der Einsatz eines Antikörpers gegen den zweiten zellgebundenen Rezeptor von TNFSF14, HVEM, zeigte hier keine Inhibition der Adipozytendifferenzierung (Fig.6). Der Antikörper war allerdings nicht als agonistisch bereits beschrieben und eine solche Aktivität wurde hier auch nicht weiter aufgeklärt. Allerdings scheint eine Aktivierung des Lymphotoxin-beta Rezeptors in den 3T3-L1 Zellen alleine hinreichend zu sein, um eine dem naturgemässen Liganden vergleichbare Inhibition der Differenzierung zu realisieren, wie in Fig. 5 gezeigt.

Auch wenn die Anwesenheit der Rezeptoren bereits durch die funktionellen Daten belegt werden konnte wurden folgende Rezeptoren auch in direkten Nachweisexperimenten belegt: Figur 4 zeigt die Analyse der Expression des Rezeptors als Target für den aktivierenden Antikörper in Maus 3T3-L1 Zellkulturen. Der Maus-HVEM-Rezeptor von TNFSF14 wurde mit dem Antikörperpaar aus Rezeptor-spezifischem Ratten IgG Antikörper (Firma Alexis Biochemicals, Katalognummer ALX-804-156) und einem sekundären Alexa-Fluor Ziege-anti-Ratte IgM spezifischen Antikörper A-21212 der Firma Molecular Probes nachgewiesen. Der Lymphotoxin-beta-Rezeptor von TNFSF14 wurde mit dem Antikörperpaar aus Rezeptor-spezifischem Ratten IgG Antikörper (Firma Alexis Biochemicals, Katalognummer ALX-804-145) und einem sekundären Alexa-Fluor Ziege-anti-Ratte IgG spezifischen Antikörper (Firma Molecular Probes, Katalognummer A-11006) nachgewiesen. Die Färbung subkonfluenter 3T3-Zellen erfolgte nach folgendem Schema: Aussaat von 200.000 Zellen in einer 6-well Schale. 24 Stunden später Färbung und Messung am FACS-Messgerät: Dazu wurden die Zellen trypsinisiert und pelletiert und anschliessend in 200 µl Pufferlösung (PBS + 1 % FCS) bzw. Pufferlösung mit Rezeptor-spezifischen Antikörper nach Herstellerangaben (1-5 µg/ml) für eine Stunde auf Eis inkubiert. Danach erfolgte eine Zugabe von 1ml PBS + 1%FCS und die Pelletierung der Zellen. Wiederaufnahme erfolgte in 200 µl PBS-Puffer mit 1% FCS in dem der sekundäre Antikörper (2µg/ml) gelöst war. Es schloss sich eine weitere Inkubation auf Eis für weitere 45 Minuten an. Die Zellen wurden anschliessend wieder mit 1ml PBS Puffer+1 % FCS versetzt und pelletiert. Nach Wiederaufnahme in 500 µl Puffer wurden die Zellen anschliessend der Messung im FACS Gerät zugeführt. Dabei wurde die Messung der Fluoreszenzintensitäten nur für lebende Zellen durchgeführt.
Das Ergebnis zeigt, dass auf den murinen 3T3-L1 Zellen der Lymphotoxin-beta Rezeptor für TNFSF14 nachgewiesen werden konnte. Überraschenderweise wurde auch der HVEM-Rezeptor für TNFSF14 nachgewiesen, der sonst als vorwiegend auf Lymphozyten-Zellen beschränkt beschrieben wurde.

### Beispiel 3:

### Untersuchungen zu möglichen adversen Effekten nach einer Rezeptoraktivierung

Die Art und Weise, wie die erfindungsgemässen Proteine die Hemmung der Lipideinlagerung in Adipozyten bewerkstelligen, kann durch weitere Analysen in den Zellkulturen näher bestimmt werden. Eine Inhibition der Differenzierung bzw. der Lipideinlagerung in differenzierenden Adipozyten kann beispielsweise durch proteinvermittelte Aktivitäten wie Zelltod, Verhinderung des Ausstiegs aus dem Zellzyklus, Blockade eines differenzierungsspezischen Genexpression oder eine Blockade der Lipogenese im engeren Sinne oder durch eine Verstärkung der Lipolyse herbeigeführt werden.

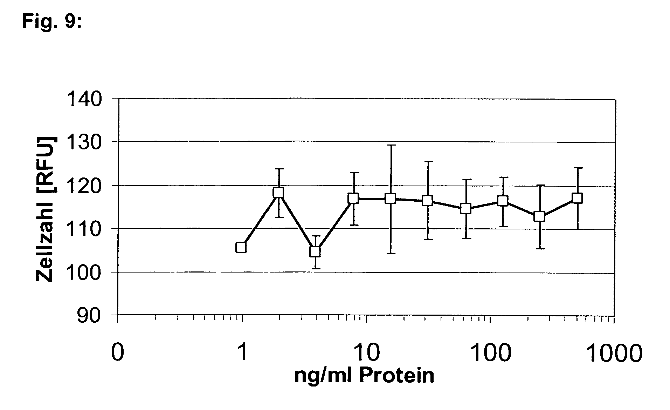

Insbesondere die Bestimmung bzw. der Ausschluss zytotoxischer Mechanismen bei der beobachteten Adipozytendifferenzierungsinhibition wurde wie folgt untersucht: Figur 9 zeigt die Bestimmung der Zellzahl von subkonfluenten 3T3-Zellkulturen. Die Zellen wurden in einer Dichte von 3.000 Zellen pro Vertiefung einer 96-well Zellkulturschale ausgesät. 24 Stunden später wurde das Wachstumsmedium der 3T3-L1 Zellen gegen Medium mit reduziertem FCS-Gehalt (0,5%) ausgewechselt. Weitere 48 Stunden später wurde ein weiterer Mediumwechsel zu 2% FCS und löslichem rekombinantem TNFSF14 durchgeführt. Dabei wurde eine Dosis-Wirkungsbeziehung über einen Konzentrationsbereich von 0,1 bis 500 ng/ml Protein untersucht. 72 Stunden nach Zugabe der Proteine in Medium mit 2% FCS wurden zu 100 µl Kulturmedium 10 µl AlamarBlue Reagenz (Fa. BioSource) zugefügt und für 4 Stunden bei 37°C inkubiert. Das AlamarBlue Reagenz stellt ein Substrat für lebende Zellen zur Verfügung, das enzymatisch umgesetzt werden kann und das Produkt kann fluorimetrisch bei einer Exzisionswellenlänge von 530 nm und einer Emissionswellenlänge von 590 nm gemessen werden. Damit ist im linearen Messbereich eine indirekte Zellzahlbestimmung gegeben. Die Ergebnisse zeigen, dass subkonfluente 3T3-L1 Zellkulturen, durch die Zugabe von rekombinant löslichem Proteinfragment von TNFSF14 (Fig.9) weder zu einer verstärkten Proliferation angeregt wurden, noch ein Zelltod messbar war. Diese Ergebnisse wurden auch an differenzierenden 3T3-L1 Zellkulturen bestätigt.

Zur Bestimmung der akuten in vivo Toxizität einer Rezeptoraktivierung kann folgendes untersucht werden: In Mäusen des Stammes C57/BI6/J wird nach Bestimmung der maximal tolerierten Dosis (MTD) a) die Verringerung der Gewichtszunahme in normalgewichtigen Mäusen (ohne vorhergehende Hochkaloriendiät) und b) die Nettoreduktion des adipösen Gewebes in zu Versuchsbeginn normalgewichtigen oder fettleibigen Mäusen (unter einsetzender oder aufrechterhaltener Hochkaloriendiät) nach Zugabe des erfindungsgemäß eingesetzten Proteins durch Wiegen oder Bestimmung des BMI ("body mass index") ermittelt. Die Bestimmung der maximal tolerierten Dosis erfolgt mit 3 Mäusen (Stamm C57BI6/J Wildtyp) durch Gabe von 0,002; 0,02; 0,2; 2,0 und 10 mg Protein/kg als einmalige intravenöse Dosis. Eine anschließende Feinjustierung der Dosis erfolgt mit je 3 Mäusen (Stamm C57BI6/J Wildtyp) durch einmalige Gabe des Proteins. In einem Dosistoleranztest wird durch wiederholte Gabe einer ausgewählten Dosis über mehrere Applikationen anhand von Blutparametern und Serumstabilität die zur Applikation notwendige Dosis ermittelt. Insbesondere wurde hier bereits gezeigt, dass die einmalige intravenöse Gabe in weibliche C57BI6 Mäuse von bis zu 10 mg/kg Körpergewicht im Falle von rekombinantem löslichem Proteinfragment von TNFSF14 zu keinerlei von der Testsubstanz verursachten klinischen Symptomen innerhalb von 48 Stunden geführt hat.

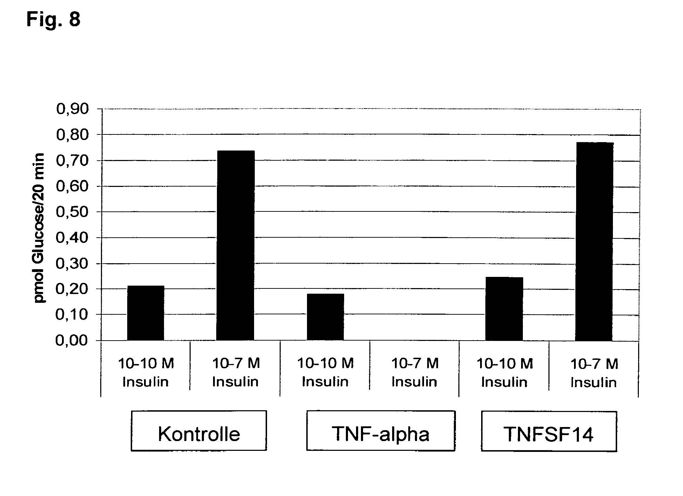

Da die Adipozytendifferenzierung ein unter anderem insulin-abhängiger Prozess ist, sollte bestimmt werden, ob die Rezeptoraktivierung zu einer negativen Modulation der Insulin-Signalgebung in Adipozyten führen kann. In diesen Experimenten, welche in Figur 8 gezeigt sind, wurden Untersuchungen zur Interferenz von TNFSF14 mit insulin-abhängiger Glukoseaufnahme in 3T3-L1 Zellen durchgeführt. 500.000 primäre Adipozyten am Tag 12 nach der Induktion zur Differenzierung wurden in einer 3 cm Schale vorgehalten und wie folgt zur Bestimmung der insulinabhängigen Glukoseaufnahme behandelt:
Versuchsansätze: Kontrollen: 1 Zellkultur für Hintergrund (0' Insulinstimulation), Ein leeres well/ Schälchen zur Bestimmung des Hintergrundes. Die Stimulation erfolgt mit 10⁻⁷, (ohne oder 10⁻¹⁰ Insulin, da 10⁻¹⁰ Insulin in diesem Zusammenhang nicht stimulierend wirkte. In jeder 3cm Schale befanden sich 800 µl Medium DMEM/Ham F12 mit 5 mM Glucose. Differenzierte Zellen wurden vier mal mit je 2 ml PBS warm waschen, dann je Schale 800 µl DMEM/F12 mit 5mM Glukose und jeweils die erfindungsgemässen Proteine zugegeben. Anschliessend erfolgte eine Inkubation für 1 Stunde bei 37 °C. Anschliessend Zugabe von Insulin. Platten wurden bei geringster Schüttelfrequenz 15 Minuten bei 37 °C inkubiert. 2-Deoxy-D-[1-H3]-Glucose (AmershamTRK 383) wurde 1:10 verdünnt in DMEM/F12. Während der Inkubationszeit wurden Röhrchen für den Szintillationszähler bereitgestellt: eines je Messpunkt und zusätzlich eines für das leere well und eines für komplette eingesetzte Menge 2-Deoxy-D-[1-H3]-Glucose. Nach 15 Minuten Inkubation wurden zu jedem well 8 µl der verdünnten 2-Deoxy-D-[1-H3]-Glucose gegeben. Platten wurden erneut für 20 Minuten unter leichtem Schütteln bei 37 °C inkubiert. Platten wurden nach 20 Minuten auf Eis gestellt. Medium wurde mit der Pumpe abgesaugt. Zellen 3 mal mit je 1 ml PBS eiskalt gewaschen. Je well 500 µl IGEPAL (Sigma I 3021) zugegeben, 20 min auf Eis lysiert. Komplettes Volumen aus jedem Well wurde in die Szinitillationscups pipettiert. Zusätzlich in ein Cup 500 µl IGEPAL plus 8 µl der verdünnten 2-Deoxy-D-[1-H3]-Glucose. In jedes Cup 3.5 ml der Szinitillationsflüssigkeit (Rotiszint eco plus, Roth, 0016.2) pipettieren. Messung im Szintillograph über den Bereich einer Minute pro Probe. Auswertung mit Hilfe des Programms Mikro Beta Windows. In der Darstellung der Ergebnisse ist die basale Glukoseaufnahme subtrahiert, so dass nur der insulinabhängige Anteil der Glukoseaufnahme gezeigt ist.

Die Rezeptoraktivierung durch TNFSF14 zeigte in diesem Versuchsaufbau keine negative Beeinflussung der insulin-abhängigen Glukoseaufnahme.

### Beispiel 4:

### Verringerung des Gewichts bzw. der Gewichtszunahme im Mausmodell

Bevorzugte Tiermodelle sind solche, die der humanen Situation der Adipositas bzw. des Typ II Diabetes in besonderer Weise entsprechen. Dafür kommen bevorzugt Nager wie zum Beispiel Mäuse oder Ratten sowie Hunde, Schweine und Primaten in Betracht. Eine besonders bevorzugte experimentelle Ausführungsform ist eine diätinduzierte Adipositas, gegebenenfalls assoziiert mit einer Insulin-Resistenz oder Typ II-Diabetes, in Mäusen oder Ratten durch Zuführung hochkalorischer Kost. Die hochkalorische Kost, das ist zum Beispiel eine >20-45 kcal%ige Fettdiät, kann dabei bereits deutlich vor oder zeitgleich mit der Gabe der erfindungsgemässen Proteine oder Fragmente bzw. der pharmazeutischen Zusammensetzung verabreicht werden.

Die Tiere können demnach zum Zeitpunkt der Gabe des Proteins bzw. Proteinfragments normal- oder bereits übergewichtig sein. Ziel ist es eine Verringerung bzw. Verhinderung einer Gewichtszunahme und/oder eine Gewichtsreduktion zu bewirken. Gegebenenfalls auch eine Verbesserung der Insulin-Sensitivität bzw. der Glukosetoleranz. Dies kann jeweils entweder unter einsetzender oder fortgesetzter hochkalorischer Diät oder unter einem Wechsel zu normal- oder hypokalorischer Diät ― für den Fall bereits übergewichtiger Tiere - erfolgen. Der Wechsel der Diäten erfolgt dabei jeweils zum Zeitpunkt der erstmaligen Gabe des Proteins bzw. Proteinfragments bzw. der pharmazeutischen Zusammensetzung.

In Mäusen des Stammes C57/B16/J wird nach Bestimmung der maximal tolerierten Dosis (MTD) a) die Verringerung der Gewichtszunahme in normalgewichtigen Mäusen (ohne vorhergehende Hochkaloriendiät) und b) die Nettoreduktion des adipösen Gewebes in zu Versuchsbeginn normalgewichtigen oder fettleibigen Mäusen (unter einsetzender oder aufrechterhaltener Hochkaloriendiät) nach Zugabe des erfindungsgemäß eingesetzten Proteins durch Wiegen oder Bestimmung des BMI ("body mass index") ermittelt.
Die Bestimmung der maximal tolerierten Dosis erfolgt mit 3 Mäusen (Stamm C57BI6/J Wildtyp) durch Gabe von 0,002; 0,02; 0,2; 2,0 und 10 mg Protein/kg als einmalige intravenöse Dosis. Eine anschließende Feinjustierung der Dosis erfolgt mit je 3 Mäusen (Stamm C57BI6/J Wildtyp) durch einmalige Gabe des Proteins. In einem Dosistoleranztest wird durch wiederholte Gabe einer ausgewählten Dosis über mehrere Applikationen anhand von Blutparametern und Serumstabilität die zur Applikation notwendige Dosis ermittelt.

Die applizierten Antikörper sind prinzipiell im Blutserum mittels ELISA nachweisbar. Dabei kann ein Absinken der Menge an detektierbarem Proteinfragment aus dem Blutserum über die Zeit angenommen werden, wie es für ein sich im Organismus verteilendes Protein zu erwarten ist. Die inhibitorische Aktivität kann in den Mausseren mittels Zugabe in den 3T3-L1 Zellkulturassay mit Nile Red-Lipidbestimmung gemessen werden. Es kann so gezeigt werden, dass die Antikörper intravenös verabreicht werden können und keine klinische Toxizität verursachen.

Im Rahmen weiterführender Experimente wird die oben gefundene Maximaldosis bis zu einer geringsten Dosis verringert, die noch einen therapeutischen Effekt bewirkt. In dem anschließenden Test zur Ermittlung der Gewichtsveränderung wird die so ermittelte Dosis parenteral (intravenös, intraperitoneal, subkutan oder intramuskulär) verabreicht. Bevorzugt sind die intraperitoneale, intravenöse oder subkutane Applikation.

Zur Ermittlung der Verringerung der Gewichtszunahme in normalgewichtigen Mäusen werden normalgewichtige Mäuse unter Normalkaloriendiät (20 kcal% Fettdiät) oder insbesondere unter hochkalorischer Diät (45% kcal%) jeweils ohne (unbehandelt) und mit Zugabe des erfindungsgemäßen Proteins (behandelt) 10-14 Wochen gehalten. Während und nach Ablauf der Versuchsreihe wird die Gewichtszunahme durch Wiegen und/oder Bestimmung des BMI ermittelt.

Bei behandelten Mäusen, die unter Normalkaloriendiät gehalten wurden, ist im Gegensatz zu unbehandelten Mäusen eine Gewichtsreduktion oder verringerte Gewichtszunahme zu beobachten. Bei behandelten Mäusen, die unter Hochkaloriendiät gehalten wurden, ist im Vergleich zu unbehandelten Mäusen ein geringerer Gewichtszuwachs bzw. eine Gewichtsreduktion zu beobachten. Diese Gewichtsreduktion kann auch auf eine verringerte Fettgewebsmasse zurückgeführt werden.

Zur Ermittlung der Verringerung der Gewichtszunahme in fettleibigen Mäusen werden fettleibige Mäuse (die zuvor unter Hochkaloriendiät (45 kcal% Fettdiät) gehalten wurden), unter Normalkaloriendiät (20 kcal% Fettdiät) und/ oder unter fortgesetzter Hochkaloriendiät jeweils ohne (unbehandelt) und mit Zugabe des erfindungsgemäßen Proteins (behandelt) 10-14 Wochen gehalten. Während und nach Ablauf der Versuchsreihe wird die Gewichtszunahme durch Wiegen und/oder Bestimmung des BMI ermittelt.

Bei behandelten Mäusen, die unter Normalkaloriendiät oder fortgesetzter Hochkaloriendiät gehalten wurden, kann eine stärkere Gewichtsreduktion gezeigt werden als bei unbehandelten Mäusen. Bei unbehandelten Mäusen unter Hochkaloriendiät ist eine weitere Gewichtszunahme zu beobachten. Bei behandelten Mäusen ist hingegen eine Gewichtsabnahme bzw. eine verringerte Gewichtszunahme festzustellen.

Insbesondere wurde hier in einem Tierexperiment folgendes gezeigt: Je acht fünf Wochen alte männliche C57BL6/J Mäuse wurden jeweils mit einem Expressionskonstrukt für rekombinantes sekretiertes und lösliches murines Proteinfragment von TNFSF14 injiziert. Zweck dieses Versuchsaufbaues war es, einen Einfluss der Proteinexpression auf die Zunahme an Körpergewicht zu beobachten. Die Tiere wurden über den gesamten Beobachtungszeitraum unter normal-kalorischer Kost gehalten.

Es wurde ein TNFSF14-Expressionskonstrukt verwendet, welches ein Fragment des Maus-Homologs enthielt, wie nachfolgend beschrieben. Dieses Fragment kodierte für eine rekombinante lösliche sekretierte murine Form von TNFSF14.
Die Expressionskonstrukte wurden erstellt, indem eine geeignete murine RNA-Quelle (Maus-Thymus RNA) mit geeigneten Primem (Umfassung der Sequenzen kodierend für: Maus-TNFSF14: Asp-72 - Val-239) eine RT-PCR durchgeführt wurde. Dabei wurden zusätzlich am 5'-Ende: 6 Histidinreste und am 3'-Ende: 2xTGA-Stop Codons und Sequenzen für die Restriktionsspaltstellen Xhol und BgIII eingeführt.
Verwendete Primer:
m14-F-His: 5'-CATCACCATCACCATCACGATGGAGGCAAAGGCTCCTG- 3' (vgl. SEQ ID NO 2)
m14-R: 5'-AGATCTCGAGTCATCAGACCATGAAAGCTCC- 3' (vgl. SEQ ID NO 3)

Das Reaktionsprodukt wurde direkt in den Vektor pSECTag/FRT/V5-His-TOPO (Invitrogen, Karlsruhe, Deutschland) nach Angaben des Herstellers für die Klonierung von PCR-Produkten eingefügt. Auf diese Weise erhielt das Proteinfragment von TNFSF14 aminoterminal im Leserahmen das Sekretionssignal aus dem Protein für die Ig-kappa-Kette gefolgt von 6 Histidinresten vorangestellt. Somit kodierte der derart generierte offene Leserahmen für die folgenden sekretierten Proteine:
Maus-TNFSF14-Leserahmen (vgl. SEQ ID NO 1)
METDTLLLWVLLLWVPGSTGDAAQPARRARRTKLALHHHHHHDGGKGSWEKLIQD QRSHQANPAAHLTGANASLIGIGGPLLWETRLGLAFLRGLTYHDGALVTMEPGYYYV YSKVQLSGVGCPQGLANGLPITHGLYKRTSRYPKELELLVSRRSPCGRANSSRVWW DSSFLGGVVHLEAGEEVVVRVPGNRLVRPRDGTRSYFGAFMV

Nachfolgend wurde der Leserahmen in den Zielvektor pBS-HCRHPI-A (Miao et al, 2003, Human Gene Therapy 14:1297-1305), bestimmt für die in-vivo Genexpression in Tieren, umkloniert: Die Exzision aus pSECTag/FRT/V5-His-TOPO-Vektoren erfolgte mit Hilfe der Restriktionsspaltstellen Nhel und BglII und Auffüllung der überstehenden Enden ― Einfügen in pBS-HCRHPI-A erfolgte über Öffnung mit dem Restriktionsenzym EcoRV und Verbinden mit den glatten Enden mit den jeweils eingefügten Fragmenten. Das resultierende Plasmid erhielt die Bezeichnungen pXAP02 (mTNFSF14).

Die Transfektion der Plasmide in C57BI6/J Mäuse, kodierend für rekombinantes sekretiertes und lösliches murines Proteinfragment von TNFSF14, erfolgte nach einem Verfahren, erstmals beschrieben von Zhang et al. (Gene Therapy 2000; 7:1344). Dabei wird durch einen hydrodynamischen Druck, erzeugt durch schnelle Injektion der plasmidhaltigen Lösung in die Schwanzvene, der Eintritt der Plasmid-DNA bevorzugt in Lebergewebe erreicht. Dort kann dann eine wochen- bis monatelange Genexpression realisiert werden. Das Verfahren in Kürze: Jeweils 50 µg endotoxinfrei- präpariertes Plasmid wurde in PBS -Lösung in einer Lösungsmittelmenge entsprechend jeweils 10% des Körpergewichts der zu injezierenden Maus mit einer Geschwindigkeit von 1ml/5 sec verabreicht. In der Folge wurden die Tiere bis 8 Wochen nach Injektion wöchentlich gewogen. An entnommenen Blutseren wurde der Gehalt an jeweils exprimiertem sekretierten löslichen murinen Proteinfragment von TNFSF14 mittels ELISA bestimmt. ELISA-Antikörperpaare für murines TNFSF14: Für die Beschichtung: anti-Penta-HIS-Antibody (Qiagen, Katalognummer #34660) und für den spezifischen Nachweis: antihLIGHT-Antibody Biotin-conjugated (R&D Systems, Katalognummer # BAF664; hat in Vorversuchen auch Kreuzreaktivität gegen Maus-TNFSF14 gezeigt.
Die Korrelation von Proteinexpression und Körpergewicht wurde untersucht.
Die Experimente beschrieben in Fig.7 haben gezeigt, dass das Ausmaß der Expression, von murinem rekombinanten sekretierten löslichen Proteinfragment von TNFSF14, mit der beobachteten Gewichtszunahme im Untersuchungszeitraum eindeutig negativ korreliert. Es ergibt sich aufgrund der vorliegenden Ergebnisse ein Korrelationskoeffizient für TNFSF14 von -0,71. Der Korrelationskoeffizient gibt an, wie strikt eine Korrelation ist. Werte zwischen -1 und +1 sind möglich. Ein Wert von 0 besagt, daß keine Korrelation vorliegt. +1 bzw. -1 sagen aus, dass eine absolut strikte (bei Vorzeichen Minus: negative) Korrelation vorliegt. Diese Korrelationen waren jeweils über den gesamten Beobachtungszeitraum von 8 Wochen zu beobachten.

Der durch die Korrelation angegebene Trend wird auch in Wiegungen von ausgewählten Fettgewebsdepots ("epidydimale fat pads" ― definierte gut messbare Fettgewebe an den Gonaden männlicher Tiere, perirenales und kolon-assoziiertes Fettgewebe) bestimmt. Die Reduktion des Körpergewichts findet hier eine Entsprechung in einer Reduktion der Gewichte ausgewählter Fettdepots.

Solche weiterführenden Experimente sind bevorzugt, in denen zum Zeitpunkt der Inhibitorgabe 12 Wochen alte C57BI6 Mäuse bis zum Zeitpunkt der Injektion mit normal kalorischer Diät oder hochkalorischer Diät und anschliessend unter hochkalorischer Diät für weitere 8 Wochen gehalten werden. Unter diesen Bedingungen einer Diät-induzierten Adipositas ist zu erwarten, dass die Präsenz der hier beschriebenen Proteinfragmente von TNFSF14 bzw. eines aktivierenden anti-LT-β-R AKs zu einer signifikant verminderten Gewichtszunahme oder auch absoluten Gewichtsreduktion führen, bevorzugt verursacht durch ein signifikant vermindertes Gewicht der Fettgewebsdepots.

## Patentansprüche

1. Verwendung eines Lymphotoxin-β-Rezeptor aktivierenden Agenz, vorzugsweise eines Lymphotoxin-β-Rezeptor-Antikörpers (anti-LT-β-R-AK), zur Herstellung einer pharmazeutischen Zusammensetzung zur Vorbeugung und/oder Behandlung von Adipositas und/oder von Adipositas-assoziierter Diabetes (Typ II) und/oder Metabolischem Syndrom und/oder anderen Adipositas-assoziierten Krankheiten.

2. Verwendung eines Lymphotoxin-β-Rezeptor aktivierenden Agenz nach Anspruch 1, wobei das Agenz eine Aktivierung des Lymphotoxin-β-Rezeptor-Signalwegs bewirkt.

3. Verwendung eines Lymphotoxin-β-Rezeptor aktivierenden Agenz nach einem der Ansprüche 1 oder 2, wobei die Aktivierung allein oder in Kombination mit anderen Lymphotoxin-β-Rezeptor aktivierenden Agenzien stattfindet.

4. Verwendung eines Lymphotoxin-β-Rezeptor aktivierenden Agenz nach einem der Ansprüche 1 bis 3, wobei das Agenz durch die rezeptorvermittelte Signaltransduktion die Differenzierung von Präadipozyten zu reifen Adipozyten und/oder die Einlagerung von Lipidmolekülen in sich differenzierenden und/oder bereits reifen Adipozyten hemmt und/oder die Lipolyse fördert.

5. Verwendung eines Lymphotoxin-β-Rezeptor aktivierenden Agenz nach einem der Ansprüche 1 bis 4, wobei das Agenz einen agonistischen anti-LT-β-R-Antikörper umfasst.

6. Verwendung eines Lymphotoxin-β-Rezeptor aktivierenden Agenz nach einem der Ansprüche 1 bis 5, wobei das Agenz gegen mindestens ein aktivierendes Epitop des Lymphotoxin-β-Rezeptors gerichtet ist.

7. Verwendung eines Lymphotoxin-β-Rezeptor aktivierenden Agenz nach einem der Ansprüche 1 bis 6, wobei das Agenz polyklonale und/oder monoklonale anti-LT-β-R-Antikörper umfasst.

8. Verwendung eines Lymphotoxin-β-Rezeptor aktivierenden Agenz nach einem der Ansprüche 1 bis 7, wobei das Agenz funktionelle Varianten und/oder Derivate von anti-LT-β-R-Antikörpem, insbesondere Moleküle mit ähnlichen Bindungseigenschaften wie chimäre, rekombinante, humanisierte oder anderweitig modifizierte Antikörper, Antikörperfragmente, Einzeldomänenantikörper und andere spezifische Bindungsproteine, umfasst.

9. Verwendung eines Lymphotoxin-β-Rezeptor aktivierenden Agenz nach Anspruch 1 bis 8, wobei das Agenz einen Agonisten, insbesondere ein Protein mit ähnlichen Bindungseigenschaften wie Antikaline, Affiline, oder ein Nicht-Protein wie Aptamere, umfasst.

10. Pharmazeutische Zusammensetzung, enthaltend ein Lymphotoxin-β-Rezeptor aktivierendes Agenz, wie in einem der Ansprüche 1 bis 9 definiert.

11. Stoff, **dadurch gekennzeichnet, dass** er mindestens eines der aktivierenden Epitope des LT-β-Rezeptors umfasst.

12. Verwendung eines Stoffs nach Anspruch 11 zur Herstellung eines aktivierenden LT-β-R-Antikörpers.

13. Pharmazeutische Zusammensetzung, enthaltend einen Stoff nach Anspruch 11.

14. Verwendung eines Stoffs nach Anspruch 11 zur Herstellung eines Impfstoffs.

15. Verwendung eines Stoffs, enthaltend ein aktivierendes Epitop des LT-β-R, zur Herstellung eines Impfstoffes zur Vorbeugung und/oder Behandlung von Adipositas und/oder von Adipositas-assoziierter Diabetes (Typ II) und/oder Metabolischem Syndrom und/oder anderen Adipositas-assoziierten Krankheiten.

16. Verfahren zur Selektion eines LT-β-R aktivierenden Agenz umfassend den Schritt Inkontaktbringen des bzw. der zu testenden Agenz(ien) mit mindestens einem Stoff enthaltend ein aktivierendes Epitop des LT-β-R und Nachweis einer Wechselwirkung des Agenz mit dem Epitop.

17. Verfahren nach Anspruch 16, **dadurch gekennzeichnet, dass** der Stoff von einer Zelle exprimiert wird und der Phänotyp der Zelle in Anwesenheit bzw. Abwesenheit des Agenz bestimmt wird.

18. Verfahren zur Selektion eines LT-β-R aktivierenden Agenz, wobei durch Bindung des Agenz an mindestens ein aktivierendes Epitop des LT-β-R die Aktivierung des LT-β-R erfolgt, und das Verfahren die folgenden Schritte umfasst:
a) Aussaat undifferenzierter Präadipozyten und Kultivieren bis zur Postkonfluenz;
b) Inkubieren der Zellen mit einem spezifischen Adipozyten-Differenzierungssubstanz-Gemisch und simultanes Inkubieren der zur Differenzierung angeregten Zellen mit zu testenden Agenzien;
c) Messen des Differenzierungsgrads der Zellen wobei dieses Messen die Detektion des intrazellulären Lipidgehalts umfasst;
d) Identifizieren des Fettzelldifferenzierung- modulierenden Agenz anhand der Abweichung der Test-Signalstärke gegenüber negativen Kontrollen;
e) Testen des in Schritt d) positiv identifizierten Agenz auf spezifische Bindung an den LT-β-R.

19. Verfahren nach einem der Ansprüche 16 bis 18, **dadurch gekennzeichnet, dass** die Zelle eine Säugerzelle, bevorzugt humanen Ursprungs, ist.

20. Verfahren nach einem der Ansprüche 16 bis 19, **dadurch gekennzeichnet, dass** die Zelle ein primärer Präadipozyt oder primärer Adipozyt ist oder von einem primären Präadipozyten oder primären Adipozyten abstammt.

21. Verfahren nach einem der Ansprüche 16 bis 20, **dadurch gekennzeichnet, dass** die Veränderung der biologischen Aktivität von Präadipozyten oder reifen Adipozyten, vorzugsweise 3T3-L1-Zellen oder humanen Präadipozyten oder Adipozyten, bestimmt wird.

22. Verfahren nach einem der Ansprüche 16 bis 21, **dadurch gekennzeichnet, dass** die Veränderung der biologischen Aktivität in einer Hemmung oder Verringerung der Fetteinlagerung in Zellen, vorzugsweise Präadipozyten, sich differenzierenden Adipozyten oder reifen Adipozyten, besteht.

23. Verfahren nach einem der Ansprüche 16 bis 22, **dadurch gekennzeichnet, dass** die Fetteinlagerung mittels Bestimmung des intrazellulären eingelagerten Lipids gemessen wird.

## Geänderte Patentansprüche

### Geänderte Patentansprüche gemäss Regel 86(2) EPÜ.

**1.** Verwendung eines agonistischen Anti-Lymphotoxin-β-Rezeptor-Antikörpers zur Herstellung einer pharmazeutischen Zusammensetzung zur Vorbeugung und/oder Behandlung von Adipositas und/oder von Adipositas-assoziierter Diabetes (Typ II) und/oder Metabolischem Syndrom und/oder anderen Adipositasassoziierten Krankheiten.

**2.** Verwendung eines agonistischen Anti-Lymphotoxin-β-Rezeptor-Antikörpers nach Anspruch 1, wobei der Antiköroer eine Aktivierung des Lymphotoxin-β-Rezeptor-Signalwegs bewirkt.

**3.** Verwendung eines agonistischen Anti-Lymphotoxin-β-Rezeptor-Antikörpers nach einem der Ansprüche 1 oder 2, wobei die Aktivierung allein oder in Kombination mit anderen Lymphotoxin-β-Rezeptor aktivierenden Agenzien stattfindet.

**4.** Verwendung eines agonistischen Anti-Lymphotoxin-β-Rezeptor-Antikörpers nach einem der Ansprüche 1 bis 3, wobei der Antikörper durch die rezeptorvermittelte Signaltransduktion die Differenzierung von Präadipozyten zu reifen Adipozyten und/oder die Einlagerung von Lipidmolekülen in sich differenzierenden und/oder bereits reifen Adipozyten hemmt und/oder die Lipolyse fördert.

**5.** Verwendung eines agonistischen Anti-Lymphotoxin-β-Rezeptor-Antikörpers nach einem der Ansprüche 1 bis 4, wobei der Antikörper gegen mindestens ein aktivierendes Epitop des Lymphotoxin-β-Rezeptors gerichtet ist.

**6.** Verwendung eines agonistischen Anti-Lymphotoxin-β-Antikörpers nach einem der Ansprüche 1 bis 5, wobei der Antikörper polyklonale und/oder monoklonale anti-LT-β-R-Antikörper umfasst.

**7.** Verwendung eines agonistischen Anti-Lymphotoxin-β-Rezeptor-Antikörpers nach einem der Ansprüche 1 bis 6, wobei der Antikörper funktionelle Varianten und/oder Derivate von anti-LT-β-R-Antiköpern, insbesondere Moleküle mt ähnlichen Bindungseigenschaften wie chimäre, rekombinante, humanisierte oder anderweitig modifizierte Antikörper, Antikörperfragmente, Einzeldomänenantikörper und andere spezirfische Bindungsproteine, umfasst.

**8.** Verwendung eines agonistischen Anti-Lymphotoxin-β-Rezeptor-Antikörpers nach Anspruch 1 bis 7, wobei der Antikörper einen Agonisten, insbesondere ein Protein mit ähnlichen Bindungseigenschaften wie Antikaline, Affiline, oder ein Nicht-Protein wie Aptamere, umfasst.

**9.** Pharmazeutische Zusammensetzung, enthaltend einen agonistischen Anti-Lymphotoxin-β-Rezeptor-Antikörpers, wie in einem der Ansprüche 1 bis 8 definiert.


SEQUENCE LISTING

<110>  Xantos Biomedicine AG

<120> Agonistische Antikörper, welche an den LT-ß-Rezeptor binden und dadurch
Adipositas assoziierte Phänotypen modulieren, und deren Verwendung in der
Therapie

<130> EP34998

<160>  3

<170>  PatentIn version 3.1

<210>  1
<211>  210
<212>  PRT
<213>  Mus musculus

<400>  1

Met Glu Thr Asp Thr Leu Leu Leu Trp Val Leu Leu Leu Trp Val Pro
1               5                   10                  15

Gly Ser Thr Gly Asp Ala Ala Gln Pro Ala Arg Arg Ala Arg Arg Thr
            20                  25                  30

Lys Leu Ala Leu His His His His His His Asp Gly Gly Lys Gly Ser
        35                  40                  45

Trp Glu Lys Leu Ile Gln Asp Gln Arg Ser His Gln Ala Asn Pro Ala
    50                  55                  60

Ala His Leu Thr Gly Ala Asn Ala Ser Leu Ile Gly Ile Gly Gly Pro
65                  70                  75                  80

Leu Leu Trp Glu Thr Arg Leu Gly Leu Ala Phe Leu Arg Gly Leu Thr
                85                  90                  95

Tyr His Asp Gly Ala Leu Val Thr Met Glu Pro Gly Tyr Tyr Tyr Val
            100                 105                 110

Tyr Ser Lys Val Gln Leu Ser Gly Val Gly Cys Pro Gln Gly Leu Ala
        115                 120                 125

Asn Gly Leu Pro Ile Thr His Gly Leu Tyr Lys Arg Thr Ser Arg Tyr
    130                 135                 140

Pro Lys Glu Leu Glu Leu Leu Val Ser Arg Arg Ser Pro Cys Gly Arg
145                 150                 155                 160

Ala Asn Ser Ser Arg Val Trp Trp Asp Ser Ser Phe Leu Gly Gly Val
                165                 170                 175

Val His Leu Glu Ala Gly Glu Glu Val Val Val Arg Val Pro Gly Asn
            180                 185                 190

Arg Leu Val Arg Pro Arg Asp Gly Thr Arg Ser Tyr Phe Gly Ala Phe
        195                 200                 205

Met Val
    210

<210>  2

<211>  38

<212>  DNA

<213>  Artificial Sequence

<220>

<223>  flankierender vorwärts Primer für die lösliche, aktive Domäne von
        murinem TNFSF14

<400>  2
catcaccatc accatcacga tggaggcaaa ggctcctg                              38

<210>  3

<211>  31

<212>  DNA

<213>  Artificial Sequence

<220>

<223>  flankierender rückwärts Primer für die lösliche, aktive Domäne vo
       n murinem TNFSF14

<400>  3
agatctcgag tcatcagacc atgaaagctc c                                     31